(19) Europäisches Patentamt
European Patent Office
Office européen des brevets

(11) EP 1 363 885 B1

(12) EUROPÄISCHE PATENTSCHRIFT

(45) Veröffentlichungstag und Bekanntmachung des Hinweises auf die Patenterteilung:
**23.05.2007 Patentblatt 2007/21**

(21) Anmeldenummer: **02714169.6**

(22) Anmeldetag: **20.02.2002**

(51) Int Cl.:
***C07D 213/38*** *(2006.01)* ***C07C 233/78*** *(2006.01)*
***C07C 211/27*** *(2006.01)* ***C07D 295/12*** *(2006.01)*
***A61K 31/4406*** *(2006.01)* ***A61K 31/137*** *(2006.01)*
***A61K 31/165*** *(2006.01)* ***A61P 29/00*** *(2006.01)*
***A61P 23/00*** *(2006.01)* ***A61P 1/12*** *(2006.01)*

(86) Internationale Anmeldenummer:
**PCT/EP2002/001765**

(87) Internationale Veröffentlichungsnummer:
**WO 2002/066432 (29.08.2002 Gazette 2002/35)**

# (54) SUBSTITUIERTE PROPAN- 1,3-DIAMIN-DERIVATE UND IHRE PHARMAZEUTISCHE VERWENDUNG

SUBSTITUTED PROPANE-1,3-DIAMINE DERIVATIVES AND THE PHARMACEUTICAL USE THEREOF

DERIVES SUBSTITUES DE PROPANE-1,3-DIAMINE ET LEUR APPLICATION PHARMACEUTIQUE

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **21.02.2001 DE 10108307**

(43) Veröffentlichungstag der Anmeldung:
**26.11.2003 Patentblatt 2003/48**

(73) Patentinhaber: **Grünenthal GmbH**
**52078 Aachen (DE)**

(72) Erfinder:
- **SUNDERMANN, Bernd**
**52066 Aachen (DE)**
- **BUSCHMANN, Helmut**
**E-08950 Esplugues de Liobregat (ES)**
- **KÖGEL, Babette-Yvonne**
**52379 Langerwehe-Hamich (DE)**
- **MERLA, Beatrix**
**52078 Aachen (DE)**
- **RISCH, Nikolaus**
**32657 Lemgo (DE)**

(56) Entgegenhaltungen:
**EP-A- 1 043 306** **EP-A- 1 043 307**
**US-A- 4 017 637**

- **B. MERLA ET AL.: "A simple and highly diastereoselective one-pot synthesis of 1,3-diamines" SYNLETT., Nr. 2, 1997, Seiten 177-178, XP002204470 THIEME VERLAG, STUTTGART., DE ISSN: 0936-5214 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 363 885 B1

Printed by Jouve, 75001 PARIS (FR)

## Beschreibung

Die vorliegende Erfindung betrifft substituierte Propan-1,3-diamin-Derivate, Verfahren zu ihrer Herstellung, sie enthaltende Arzneimittel und pharmazeutische Zusammensetzungen sowie ihre Verwendung zur Herstellung von Arzneimitteln zur Behandlung und/oder Prophylaxe von Schmerz, Harninkontinenz, Juckreiz, Tinnitus aurium und/oder Diarrhoe.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist.

Klassische Opioide wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Der vorliegenden Erfindung liegt als Aufgabe zugrunde, analgetisch wirksame Verbindungen zur Verfügung zu stellen, die sich zur Schmerztherapie - insbesondere auch zur Therapie chronischer und neuropathischer Schmerzen - eignen. Darüber hinaus sollten diese Substanzen möglichst keine der Nebenwirkungen, die üblicherweise bei der Anwendung von Opioiden mit μ-Rezeptoraffinität wie Morphin auftreten, wie z.B. Übelkeit, Erbrechen, Abhängigkeit, Atemdepression oder Obstipation, hervorrufen.

Diese Aufgabe wird durch die Verbindungen der allgemeinen Struktur (I) gelöst, die analgetisch wirksam sind. Bei den erfindungsgemäßen Verbindungen handelt es sich um substituierte 1,3-Propan-diamin-Derivate der allgemeinen Struktur (I) sowie ihre pharmazeutisch akzeptablen Salze

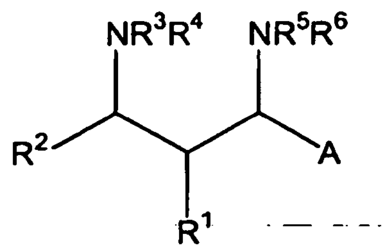

I ,

worin

| | |
|---|---|
| $R^1$ | Methyl oder Ethyl bedeutet, |
| $R^2$ | Methyl, Ethyl oder Phenyl bedeutet, |
| | oder |
| $R^1$ und $R^2$ | zusammen $-(CH_2)_4-$ bilden; |
| $R^3$ | H, n-Propyl, $-CH_2$-Phenyl oder C(=O)-$R^7$ bedeutet; |
| $R^4$ | H bedeutet; |
| $R^5$ und $R^6$ | jeweils Methyl bedeuten oder zusammen $-(CH_2)_2-O-(CH_2)_2-$ bilden; |
| A | Phenyl, 2-Chlorphenyl, 2-Methoxyphenyl, 2-Nitrophenyl oder Pyridin-3-yl bedeutet; und |
| $R^7$ | Methyl, Phenyl, 2-Fluorphenyl, 2-Chlorphenyl oder 2-Methylphenyl bedeutet. |

Die Verbindungen der allgemeinen Struktur (I) können als Racemat, in Form eines oder mehrerer ihrer Diastereomeren oder eines oder mehrerer ihrer Enantiomeren vorliegen.

Folgende Verbindungen der allgemeinen Struktur (1) sind im Stand der Technik bereits bekannt (Synlett (1997), 177-178), ohne daß deren Verwendung in einem Arzneimittel oder zur Herstellung eines Arzneimittels zur Therapie und/oder Prophylaxe von Schmerz, Harninkontinenz, Juckreiz, Tinnitus aurium und/oder Diarrhoe beschrieben wird: N, N-Dimethyl-[phenyl-(2-pyrrolidin-1-yl-cyclohexyl)-methyl]-amin, N,N-Dimethyl-[(2-morpholin-4-yl-cyclohexyl)-phenylmethyl]-amin, 4-[Phenyl-(2-pyrrolidin-1-yl-cyclohexyl)-methyl]-pyrrolidin, 4-[Phenyl-(2-pyrrolidin-1-yl-cyclohexyl)-methyl]-morpholin, 1-[Phenyl-(2-pyrrolidin-1-yi-cyclohexyl)-methyl]-piperidin, 1-[2-Methyl-1-(2-pyrrolidin-1-yl-cyclohexyl)-propyl]-piperidin, N,N-Dimethyl-(2-methyl-1,3-diphenyl-3-pyrrolid in-1-yl-propyl)-amin, N,N-Dimethyl-(2-methyl-1,3-diphenyl-3-(N,N-diethylamino)-propyl)-amin, 4-(1,3-Diphenyl-3-pyrrolidin-1-yl-propyl)-morpholin, N,N-Dimethyl-(2-methyl-1-phenyl-3-(morpholin-4-yl)-pentyl)-amin, Benzyl-[2-(dimethylamino-phenyl-methyl) cyclohexyl]-amin, (2-Methyl-1,3-diphenyl-3-piperidin-1-yl-propyl)-propylamin. Diese Verbindungen sind daher insoweit ebenfalls Gegenstand der vorliegenden Erfindung, als erfindungsgemäße Verfahren zu ihrer Herstellung, sie enthaltende Arzneimittel sowie ihre Verwendung zur Herstellung von Medikamenten zur Behandlung und/oder Prophylaxe von Schmerz, Harninkontinenz, Juckreiz, Tinnitus aurium und/oder Diarrhoe betroffen sind.

Pharmazeutisch annehmbare (akzeptable) Salze im Sinne dieser Erfindung sind solche Salze der erfindungsgemäßen Verbindungen gemäß der allgemeinen Struktur (I), die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden.

Vorzugsweise werden die pharmazeutisch annehmbaren Salze der erfindungsgemäßen Verbindungen mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Bei den gebildeten Salzen handelt es sich u. a. um Hydrochloride, Hydrobromide, Phosphate, Carbonate, Hydrogencarbonate, Formiate, Acetate, Oxalate, Succinate, Tartrate, Fumarate, Citrate und Glutaminate. Ebenfalls bevorzugt sind Solvate und insbesondere die Hydrate der erfindungsgemäßen Verbindungen, die z.B. durch Kristallisation aus wäßriger Lösung erhalten werden können.

Die erfindungsgemäßen Verbindungen der allgemeinen Struktur (I) weisen stets mindestens drei Asymmetriezentren auf, die in der untenstehenden Formel mit * gekennzeichnet sind:

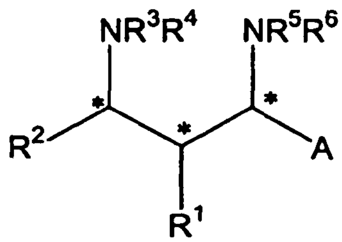

I .

Die erfindungsgemäßen Verbindungen der allgemeinen Struktur (I) können somit als Racemat, in Form eines oder mehrerer ihrer Diastereomeren, d.h. diastereomerenrein oder als Mischung zweier oder mehrerer Diastereomeren, oder in Form eines oder mehrerer ihrer Enantiomeren vorliegen, d.h. enantiomerenrein oder als nichtracemische Mischung von Enantiomeren, und zwar sowohl in Substanz als auch als pharmazeutisch annehmbare Salze dieser Verbindungen. Die Mischungen können in jedem beliebigen Mischungsverhältnis der Stereoisomeren vorliegen.

Dabei ist es bevorzugt, daß die erfindungsgemäßen Verbindungen der allgemeinen Formel (I), oder eines ihrer pharmazeutisch annehmbaren Salze, als Diastereomeren der Formel (syn,anti-I)

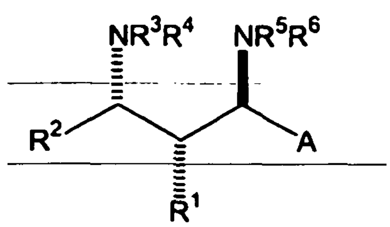

**syn,anti-I**

und gegebenenfalls enantiomerenrein vorliegen. Die für die Kennzeichnung der relativen Konfiguration (relativen Stereochemie) gewählte Bezeichnung "syn,anti" ist so zu verstehen, daß die beiden benachbarten Substituenten $NR^3R^4$ und $R^1$ in der oben gezeigten Konformation in die gleiche Raumhälfte weisen (= "syn"), während die beiden benachbarten Substituenten $R^1$ und $NR^5R^6$ in der gezeigten Konformation in entgegengesetzte Raumhälften (= "anti") weisen (S. Masamune et al., J. Am. Chem. Soc. (1982) 104, 5521-5523).

Ebenfalls bevorzugt sind Verbindungen der allgemeinen Struktur (I) bzw. ihre pharmazeutisch annehmbaren

Salze, die als Diastereomeren der Formel (anti,anti-I)

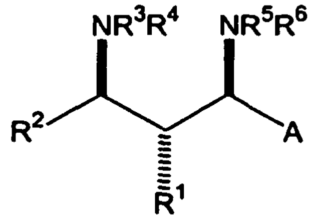

**anti,anti-I**

und gegebenenfalls enantiomerenrein vorliegen. Die für die Kennzeichnung der relativen Stereochemie gewählte Bezeichnung "anti,anti" ist so zu verstehen, daß die beiden benachbarten Substituenten $NR^3R^4$ und $R^1$ in der gezeigten Konformation ebenso in entgegengesetzte Raumhälften weisen (= "anti"), wie die beiden benachbarten Substituenten $R^1$ und $NR^5R^6$.

Außerdem bevorzugt sind Verbindungen der allgemeinen Struktur (I) bzw. ihre pharmazeutisch annehmbaren Salze, die als Diastereomeren der Formel (anti,syn-I)

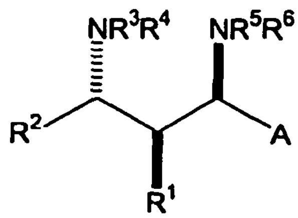

**anti,syn-I**

und gegebenenfalls enantiomerenrein vorliegen. Die für die Kennzeichnung der relativen Stereochemie gewählte Bezeichnung "anti,syn" ist so zu verstehen, daß die beiden benachbarten Substituenten $NR^3R^4$ und $R^1$ in der gezeigten Konformation in entgegengesetzte Raumhälften weisen (= "anti"), während die beiden benachbarten Substituenten $R^1$ und $NR^5R^6$ in der gezeigten Konformation in die gleiche Raumhälfte (= "syn") weisen.

Ferner sind Verbindungen der allgemeinen Struktur (I) bzw. ihre pharmazeutisch annehmbaren Salze bevorzugt, die als Diastereomeren der Formel (syn,syn-I)

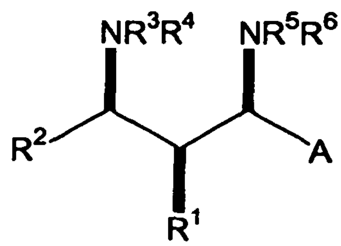

**syn,syn-I**

und gegebenenfalls enantiomerenrein vorliegen. Die für die Kennzeichnung der relativen Stereochemie gewählte Bezeichnung "syn,syn " ist so zu verstehen, daß die beiden benachbarten Substituenten $NR^3R^4$ und $R^1$ in der gezeigten Konformation ebenso in die gleiche Raumhälfte weisen (= "syn") wie die beiden benachbarten Substituenten $R^1$ und $NR^5R^6$.

Beispielhafte und vorteilhafte Verbindungen der vorliegenden Erfindung sind aus der Gruppe ausgewählt, die

- (syn,syn)-N-[2-(Dimethylaminopyridin-3-ylmethyl)cyclohexyl]-benzamid oder sein Hydrochlorid
- (syn,syn)2-(Dimethylaminopyridin-3-ylmethyl)cyclohexylamin oder sein Hydrochlorid
- (syn,syn)-N-[2-(Dimethylaminopyridin-3-ylmethyl)cyclohexyl]-2-fluorbenzamid oder sein Hydrochlorid
- (syn,syn)-2-Chlor-N-[2-(Dimethylaminopyridin-3-ylmethyl)cyclohexyl]-benzamid oder sein Hydrochlorid
- (anti,anti)-N-[2-(Dimethylaminopyridin-3-ylmethyl)cyclohexyl]-benzamid oder sein Hydrochlorid
- (anti,anti)-2-(Dimethylaminopyridin-3-ylmethyl)cyclohexylamin oder sein Hydrochlorid
- (anti,anti)-N-[2-(Dimethylaminopyridin-3-ylmethyl)cyclohexyl]-2-fluorbenzamid oder sein Hydrochlorid
- (anti,anti)-2-Chlor-N-[2-(dimethylaminopyridin-3-ylmethyl)cyclohexyl]benzamid oder sein Hydrochlorid
- (anti,anti)-N-[2-(Dimethylaminopyridin-3-ylmethyl)cyclohexyl]-2-methylbenzamid oder sein Hydrochlorid
- (syn,syn)-N-[2-(Dimethylaminopyridin-3-ylmethyl)cyclohexyl]-2-methylbenzamid oder sein Hydrochlorid
- (syn,syn)-N-[2-(Dimethylaminopyridin-3-ylmethyl)cyclohexyl]acetamid oder sein Hydrochlorid
- (anti,anti)-N-[2-(Dimethylaminopyridin-3-ylmethyl)cyclohexyl]acetamid oder sein Hydrochlorid
- (syn,syn)-N-[2-(Dimethylaminophenylmethyl)cyclohexyl]-2-fluorbenzamid oder sein Hydrochlorid
- (syn,syn)-2-(Dimethylaminophenylmethyl)cyclohexylamin oder sein Hydrochlorid
- (syn,syn)-N-[2-(Dimethylamino-phenyl-methyl)-cyclohexyl]-acetamid oder sein Hydrochlorid
- (syn,syn)-N-[2-(Dimethylamino-phenyl-methyl)-cyclohexyl]-benzamid oder sein Hydrochlorid
- (syn,syn)-2-Chlor-N-[2-(dimethylamino-phenyl-methyl)-cyclohexyl]-benzamid oder sein Hydrochlorid
- (syn,syn)-N-[2-(Dimethylamino-phenyl-methyl)-cyclohexyl]-2-methyl-benzamid oder sein Hydrochlorid
- (anti,anti)-N-[2-(Dimethylamino-phenyl-methyl)-cyclohexyl]-acetamid oder sein Hydrochlorid
- (anti,anti)-2-(Dimethylamino-phenyl-methyl)-cyclohexylamin oder sein Hydrochlorid
- (anti,anti)-N-[2-(Dimethylamino-phenyl-methyl)-cyclohexyl]-benzamid oder sein Hydrochlorid
- (anti,anti)-N-[2-(Dimethylamino-phenyl-methyl)-cyclohexyl]-2-methyl-benzamid oder sein Hydrochlorid
- (syn,syn)-2-Chlor-N-{2-[(2-chlor-phenyl)-dimethylamino-methyl]-cyclohexyl}-benzamid oder sein Hydrochlorid
- (syn,syn)-2-[(2-Chlor-phenyl)-dimethylamino-methyl]-cyclohexylamin oder sein Hydrochlorid
- (anti,anti)-2-Chlor-N-{2-[(2-chlor-phenyl)-dimethylamino-methyl]-cyclohexyl}-benzamid oder sein Hydrochlorid
- (anti,anti)-2-[(2-Chlor-phenyl)-dimethylamino-methyl]-cyclohexylamin oder sein Hydrochlorid
- (syn,syn)-N-{2-[(2-Chlor-phenyl)-dimethylamino-methyl]-cyclohexyl}-2-fluor-benzamid oder sein Hydrochlorid
- (anti,anti)-N-{2-[Dimethylamino-(2-nitro-phenyl)-methyl]-cyclohexyl}-benzamide oder sein Hydrochlorid
- (anti,anti)-2-[Dimethylamino-(2-nitro-phenyl)-methyl]-cyclohexylamin oder sein Hydrochlorid
- (anti,anti)-N-{2-[Dimethylamino-(2-nitro-phenyl)-methyl]-cyclohexyl}-2-fluor-benzamid oder sein Hydrochlorid
- (ant,anti)-2-Chlor-N-{2-[dimethylamino-(2-nitro-phenyl)-methylcyclohexyl}-benzamid oder sein Hydrochlorid
- (anti,anti)-{2-[Dimethylamino-(2-nitro-phenyl)-methyl]-cyclohexyl}-2-methyl-benzamid oder sein Hydrochlorid
- (syn,syn)-N-{2-[Dimethylamino-(2-nitro-phenyl)-methyl]-cyclohexyl}-acetamid oder sein Hydrochlorid
- (syn,syn)-N-2-[Dimethylamino-(2-nitro-phenyl)-methyl]-cyclohexylamin oder sein Hydrochlorid
- (anti,anti)-N-{2-[(2-Chlor-phenyl)-dimethylamino-methyl]-cyclohexyl}-acetamid oder sein Hydrochlorid
- (syn,anti)-2-(Dimethylamino-phenyl-methyl)-cyclohexylamin
- (syn,anti)-N-[2-(Dimethylamino-phenyl-methyl)-cyclohexyl]-benzamid
- (anti,anti)-N-{2-[Dimethylamino-(2-methoxy-phenyl)-methyl]-cyclohexyl}-benzamid
- (anti,anti)-N-{2-[Dimethylamino-(2-nitro-phenyl)-methyl]-cyclohexyl}-benzamid
- (anti,anti)-N-{2-[(2-Chlor-phenyl)-dimethylamino-methyl]-cyclohexyl}-benzamid
- (anti,anti)-N-{2-[Dimethylamino-(2-methoxy-phenyl)-methyl]-cyclohexyl}-acetamid
- (anti,anti)-2-[Dimethylamino-(2-methoxy-phenyl)-methyl]-cyclohexylamin
- (anti,anti)-N-{-2-[(2-Chlor-phenyl)-dimethylamino-methyl]-cyclohexyl}-acetamid
- (anti,anti)-2-[(2-chlor-phenyl)-dimethylamino-methyl]-cyclohexylamin
- (anti,anti)-N-{2-[Dimethylamino-(2-nitro-phenyl)-methyl]-cyclohexyl}-acetamid
- (anti,anti)-2-[Dimethylamino-(2-nitro-phenyl)-methyl]-cyclohexylamin
- (syn,syn)-2-(Dimethylamino-phenyl-methyl)-cyclohexylamin
- (syn,syn)-2-[(2-Chlor-phenyl)-dimethylamino-methyl]-cyclohexylamin
- (anti,anti)-2-Chlor-N-(3-dimethylamino-1-ethyl-2-methyl-3-phenylpropyl)-benzamid
- (anti,anti)-3-Dimethylamino-1-ethyl-2-methyl-3-phenyl-propylamin
- (syn,anti)-2-(Dimethylamino-phenyl-methyl)-cyclohexyl-N-(n-propyl)-amin
- (syn,anti)-2-(Morpholin-4-yl-phenyl-methyl)-cyclohexyl-N-(n-propyl)-amin
- (syn,anti)-2,N,N-Trimethyl-1,3-diphenyl-N'-propyl-propan-1,3-diamin
- (syn,anti)-2-(Dimethylamino-phenyl-methyl)-cyclohexyl-N-benzyl-amin
- (syn,anti)-2-(Morpholin-4-yl-phenyl-methyl)-cyclohexyl-N-benzyl-amin
- (syn,anti)-2,N,N-Trimethyl-1,3-diphenyl-N'-benzyl-propan-1,3-diamin
- (syn,anti)-2-(Dimethylamino-phenyl-methyl)-cyclohexylamin
- (syn,anti)-2-(Morpholin-4-yl-phenyl-methyl)-cyclohexylamin

- (syn,anti)-2,N,N-Trimethyl-1,3-diphenyl-propan-1,3-diamin
- (syn,anti)-2-[(2-Chlorphenyl)-dimethylamino-methyl]-cyclohexylamin
- (anti,anti)-2-[(2-Chlorphenyl)-dimethylamino-methyl]-cyclohexylamin
- (syn,syn)-2-(Dimethylamino-phenyl-methyl)-cyclohexylamin
- (anti,anti)-2-(Dimethylamino-phenyl-methyl)-cyclohexylamin
- (syn,syn)-2-[(2-Chlorphenyl)-dimethylamino-methyl]-cyclohexylamin
- (syn,syn)-2-(Dimethylamino-pyridin-3-yl-methyl)-cyclohexylamin
- (anti,anti)-2-(Dimethylamino-pyridin-3-yl-methyl)cyclohexylamin
- (syn,syn)-2-(Dimethylamino-(2-methoxyphenyl)-methyl)-cyclohexylamin
- (anti,anti)-2-(Dimethylamino-(2-methoxyphenyl)-methyl)-cyclohexylamin
- (syn,syn)-2-(Dimethylamino-(2-nitrophenyl)-methyl)-cyclohexylamin
- (anti,anti)-2-(Dimethylamino-(2-nitrophenyl)-methyl)-cyclohexylamin

umfaßt.

Ein weiterer Gegenstand der vorliegenden Erfindung sind Verfahren zur Herstellung der Verbindungen der allgemeinen Struktur (I). So können Verbindungen der allgemeinen Struktur (I), in denen $R^3$ für H, n-Propyl oder -$CH_2$-Phenyl steht und $R^4$ für Wasserstoff steht, durch Reduktion des korrespondierenden Imins der allgemeinen Formel (II)

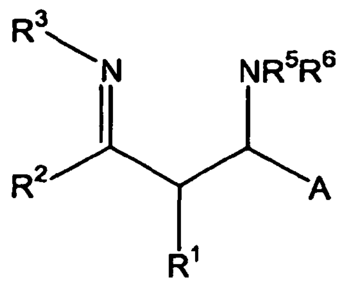

II ,

erhalten werden. Geeignete Reduktionsmittel sind beispielsweise komplexe Hydride, wie z.B. $ZnCNBH_3$, das in situ durch Umsetzung von Natriumcyanoborhydrid mit wasserfreiem Zink-(II)-chlorid in einem wasserfreien organischen Lösungsmittel gebildet werden kann, Diisobutylaluminiumhydrid (= DIBAH, DIBAL), L-Selectride (d.h. Lithium-tri-sec.-butylborhydrid) und $LiBH_4$, $NaBH_4$, $NaBH_3CN$ und $NaBH(OC(=O)CH_3)_3$. Die Reduktion erfolgt dabei bei Temperaturen von - 70 °C bis +65 °C, bevorzugt 0 °C bis +40 °C, über einen Zeitraum von 0,5 h bis 24 h. Dieses Imin-Reduktionsverfahren liefert das Diamin (I) im allgemeinen als ein Gemisch verschiedener denkbarer Stereoisomeren (Diastereomerengemisch). Alternativ kann die Reduktion auch mit Wasserstoff (bei einem $H_2$-Partialdruck von 1 bis 50 bar) in Gegenwart eines geeigneten Übergangsmetall-Katalysators, z.B. Ni, Pd, Pt oder $PtO_2$, bevorzugt in situ, ausgeführt werden.

Überraschenderweise wurde gefunden, daß das oben dargelegte Imin-Reduktionsverfahren auch zur diastereoselektiven Synthese von (anti,anti-I) bzw. (syn,syn-I) (mit $R^3$ und $R^4$ = H) angepaßt werden kann: Wird ein Imin (II) mit der relativen Konfiguration anti

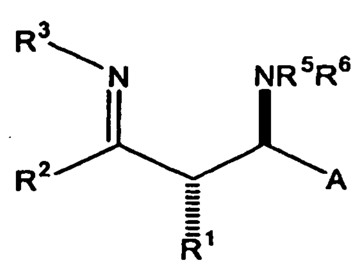

anti-II

mit einem geeigneten Reduktionsmittel, insbesondere Zinkcyanoborhydrid, $LiBH_4$, $NaBH_4$, $NaBH_3CN$ oder $NaBH(OC(=O)CH_3)_3$ in einem alkoholischen Lösungsmittel umgesetzt, wird mit hoher Stereoselektivität das Diamin (1) mit der

relativen Konfiguration anti,anti

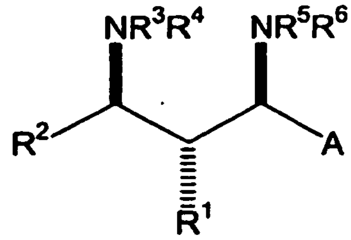

**anti,anti-I**

erhalten. Vorzugsweise wird die Reduktion in Methanol unter langsamem Erwärmen von 0 °C auf Raumtemperatur über 8 bis 24 h, insbesondere 10 bis 14 h, durchgeführt.

Wird hingegen das Imin (anti-II) mit einem geeigneten Reduktionsmittel in einem etherischen Lösungsmittel umgesetzt, so wird nahezu ausschließlich das Diamin (I) mit der relativen Konfiguration syn,syn erhalten:

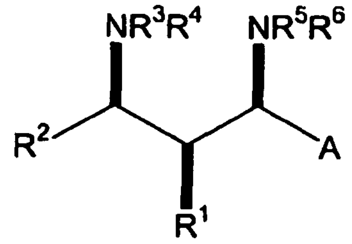

**syn,syn-I**

Vorzugsweise wird diese Reduktion mit L-Selectride oder Diisobutylaluminiumhydrid (DIBAH), insbesondere in THF und unter Erwärmen von 0 °C bis Raumtemperatur über 8 bis 24 h, insbesondere 10 bis 14 h, durchgeführt.

Zur Gewinnung der Diastereomeren des Diamins (I) mit der relativen Konfiguration syn,anti bzw. anti,syn kann das Diastereomerenproduktgemisch des nicht stereoselektiv geführten Imin-Reduktionsverfahrens beispielsweise einer fraktionierten Kristallisation, auch ihrer Salze, oder einer chromatographischen Trennung unterworfen werden.

Die im erfindungsgemäßen nicht stereoselektiven Imin-Reduktionsverfahren eingesetzten Imine der Formel (II) sind aus den entsprechenden Mannich-Basen der allgemeinen Struktur (III)

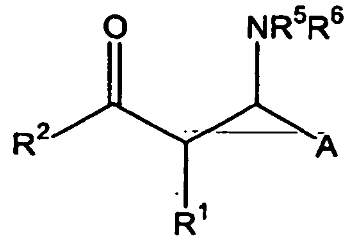

**III** ,

worin $R^1$, $R^2$, $R^5$, $R^6$ und A wie für Formel (I) und (II) definiert sind, durch Umsetzung mit Ammoniak oder einem äquivalenten Reagenz (wenn $R^3$ in Formel (II) H bedeutet) bzw. mit einem primären Amin $R^3NH_2$ (wenn $R^3$ in (II) nicht H, sondern n-Propyl oder-$CH_2$-Phenyl bedeutet) ohne weiteres zugänglich. Für den Fall, daß $R^3$ = H, ist es bevorzugt, die Mannich-Base (III) mit Ammoniumacetat in einem etherischen oder alkoholischen Lösungsmittel zu dem Imin (II)

umzusetzen, das seinerseits, vorzugsweise in situ, zur erfindungsgemäßen Verbindung (I) reduziert wird. So kann die Umsetzung von (III) mit Ammoniumacetat in wasserfreiem Tetrahydrofuran (THF) bei Temperaturen von 0 °C bis 80 °C, vorzugsweise bei 20 bis 25 °C und mit einer Reaktionszeit von 0,5 h bis 12 h, vorzugsweise von 30 min bis 120 min, insbesondere 60 min, ausgeführt werden, insbesondere wenn die anschließende Reduktion in THF ausgeführt wird. Alternativ kann die Umsetzung von (III) mit Ammoniumacetat auch in wasserfreiem Methanol bei Temperaturen von 0 °C bis 80 °C, vorzugsweise bei 20 bis 25 °C und mit einer Reaktionszeit von 0,5 h bis 12 h, vorzugsweise von 30 min bis 120 min, insbesondere 60 min, ausgeführt werden, insbesondere wenn die anschließende Reduktion in Methanol ausgeführt wird.

Analog sind die anti-konfigurierten Imine (anti-II) ausgehend von den entsprechenden anti-konfigurierten Mannich-Basen (anti-III)

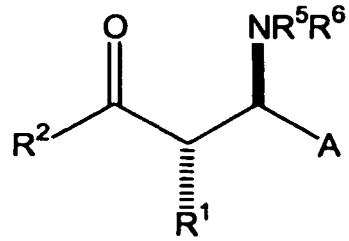

**anti-III**

zugänglich, indem diese mit dem primären Amin $R^3NH_2$ bzw. mit Ammoniak oder einem äquivalenten Reagenz, wie z.B. Ammoniumacetat, unter den für die Bildung von (II) oben angegebenen Bedingungen umgesetzt werden.

Die Herstellung der Mannichbasen (III) ist an sich literaturbekannt und wird z.B. in den Patentanmeldungen EP 1 043 307 A2 und EP 1 043 306 A2, die hiermit in die Offenbarung der vorliegenden Erfindung einbezogen werden, ausführlich dargestellt. So führt die 1,4-Addition sekundärer Amine $HNR^5R^6$ an Enone der allgemeinen Struktur (XI) - die ihrerseits durch Aldolkondensation von Ketonen der Formel (IX) mit Aldehyden der allgemeinen Formel (X) erhalten werden - zu den gewünschten Mannich-Basen (II) (US-Patent 4,017,637), die in der Regel als Gemisch der Stereoisomeren anfallen.

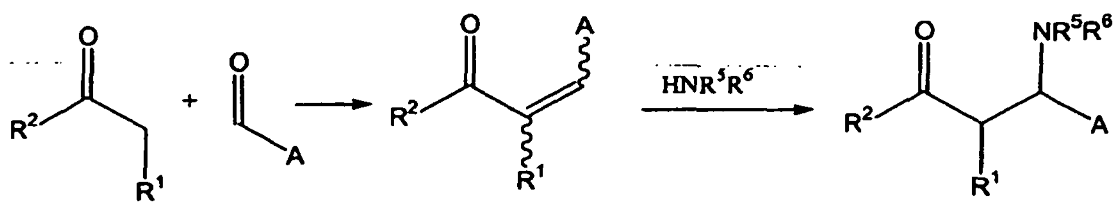


IX X XI III

Die Bedeutung der Reste $R^1$, $R^2$, $R^5$, $R^6$ und A entspricht der Bedeutung für die Formeln (I) und (II).

Die so gewonnenen Mannich-Basen (III) können als Gemisch der Stereoisomeren verwendet oder unter Einsatz von im Stand der Technik wohlbekannten Verfahren, wie z.B. Kristallisation oder Chromatographie, in ihre Diastereoisomeren aufgetrennt und als solche umgesetzt werden.

Alternativ können Mannich-Basen mit vorzugsweise anti-Konfiguration diastereoselektiv durch Umsetzung von Enaminen der allgemeinen Struktur (XII), worin die Reste R z.B. Alkyl bedeuten oder zusammen $-(CH_2)_4-$ oder $-(CH_2)_5-$ bilden, mit Iminiumsalzen der allgemeinen Struktur (VIII), in der $Z^\ominus$ ein geeignetes Gegenion, wie z.B. $Cl^\ominus$, $Br^\ominus$, $I^\ominus$ oder $AlCl_4^\ominus$, bedeutet, hergestellt werden (EP 1 043 307 A2 und EP 1 043 306 A2).

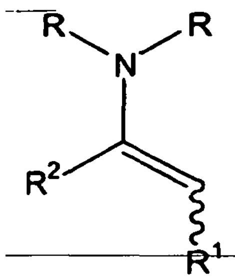

XII

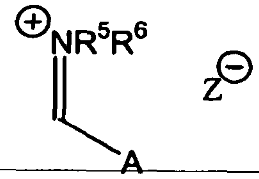

VIII

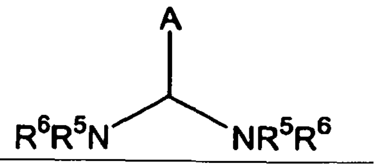

XIII

Die Enamine werden nach literaturbekannten Verfahren aus Ketonen der allgemeinen Struktur (IX) und sekundären Aminen, z.B. Dimethylamin, Pyrrolidin, Piperidin oder Morpholin, hergestellt (Acta Chem. Scand. B 38 (1984) 49-53). Die Iminiumsalze (VIII) werden nach literaturbekannten Verfahren z.B. durch Umsetzung von Aminalen der allgemeinen Struktur (XIII) mit Säurechloriden, z.B. Acetylchlorid oder Thionylchlorid (Houben-Weyl - Methoden der Organischen Chemie, E21 b (1995) 1925-1929), oder durch Umsetzung von Aldehyden der Formel (X) mit sekundären Aminen in Gegenwart von Natriumiodid, Trimethylsilyliodid und Triethylamin hergestellt (Synlett (1997) 974-976). Die Iminiumsalze (VIII) müssen dabei nicht isoliert werden, sondern können auch in situ erzeugt und mit den Enaminen der Formel (XII) bevorzugt zu den anti-Mannich-Basen (anti-III) umgesetzt werden (Angew. Chem. 106 (1994) 2531-2533). Es ist auch möglich, Ketone der allgemeinen Struktur (IX) direkt mit Iminiumsalzen (VIII) zu Mannich-Basen (III) umzusetzen. Auch in diesem Fall werden bevorzugt die Mannich-Basen (anti-III) mit anti-Konfiguration gebildet.

Aus den anti-konfigurierten Mannich-Basen (anti-III) können, falls erforderlich, auch die entsprechenden syn-konfigurierten Isomeren (syn-III) dadurch gewonnen werden, daß die Mannich-Base (anti-III) in einem geeigneten Lösungsmittel, z.B. einem Alkohol, wie Methanol oder Ethanol, oder Wasser, gelöst, mit einer hinreichend starken Säure, z.B. wäßrige Salzsäure, verdünnte Schwefelsäure oder konz. Essigsäure, versetzt und etwa 8 bis 24 h gerührt wird; dabei ist es für die gewünschte Epimerisierung wesentlich, daß die gelöste Mannich-Base (III) nicht aus der Lösung ausfällt oder auskristallisiert, sondern in Lösung bleibt. Nach dem Entfernen des Lösungsmittels werden die anti-Mannich-Base (anti-III) und die syn-Mannich-Base (syn-III) als Diastereomerengemisch, zumeist in einem Verhältnis von 1 :1, erhalten, das nach herkömmlichen Methoden (Kristallisation, Chromatographie) aufgetrennt werden kann.

Ein weiteres erfindungsgemäßes Verfahren zur Herstellung der erfindungsgemäßen Verbindungen der allgemeinen Struktur (I); in denen $R^3$ und $R^4$ jeweils H bedeuten, geht von einem Aminoalkohol der allgemeinen Struktur (IV) aus, der in einem Verfahrensschritt (a) in das korrespondierende Mesylat bzw. Tosylat der Formel (V), worin L Mesyl ($CH_3SO_2$-) oder Tosyl (4-$CH_3$-Phenyl-$SO_2$-) bedeutet, beispielsweise durch Umsetzung von (IV) mit Mesylchlorid ($CH_3SO_2Cl$) bzw. Tosylchlorid (p-Toluolsulfonsäurechlorid, 4-$CH_3$-Phenyl-$SO_2Cl$) in Gegenwart einer Base (z.B. Triethylamin), überführt wird; anschließend wird das Mesilat bzw. Tosylat (V) in einem Verfahrensschritt (b) beispielsweise mit Natriumazid zu dem Azid (VI) umgesetzt, welches in einem Verfahrensschritt (c) unter Reduktion in das erfindungsgemäße Diamin der Formel (I) überführt wird. Die Reduktion erfolgt hierbei nach literaturbekannten Verfahren, z.B. mit Natriumborhydrid in Gegenwart katalytischer Mengen Cobalt-(II)-bromid (D. M. Tschaen et al., J. Org. Chem. (1995) 60, 4324-4330) oder mit Lithiumaluminiumhydrid in Diethylether.

Dieses Verfahren kann auch so angewandt werden, daß eine erfindungsgemäße Verbindung der Formel (I) bevorzugt in einer bestimmten relativen Konfiguration erhalten wird. Geht man von einem Aminoalkohol der allgemeinen Struktur (anti,anti-IV) - ein Aminoalkohol (IV) mit der relativen Konfiguration (anti,anti) - aus, so verläuft der Verfahrensschritt (a') bevorzugt unter Erhalt der relativen Stereochemie zu der Verbindung (anti,anti-V), während die anschließende Azidbildung (b') unter Inversion der Konfiguration des Stereozentrums am O-L-Kohlenstoff Atom verläuft und somit das Azid (syn,anti-V1) ergibt. Die anschließende Reduktion von (syn,anti-VI) ergibt das Diamin (syn,anti-I).

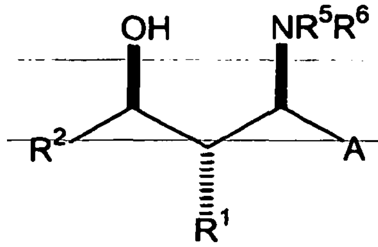

**anti,anti-IV**

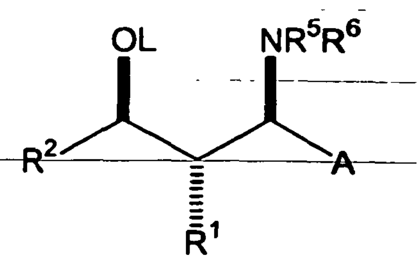

**anti,anti-V**

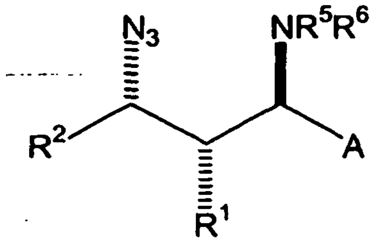

**syn,anti-VI**

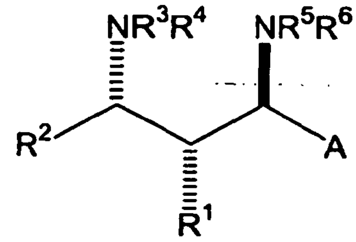

**syn,anti-I**

Entsprechend ist auch das Diamin (anti,anti-I) stereoselektiv zugänglich, wenn man das erfindungsgemäße Verfahren mit einem Aminoalkohol der allgemeinen Struktur (syn.anti-IV) beginnt und über das Mesylat bzw. Tosylat der allgemeinen Struktur (syn,anti-V) zu dem Azid der allgemeinen Struktur (anti,anti-VI) führt, das abschließend zum Diamin (anti,anti-1) reduziert wird.

Die in diesem Verfahren eingesetzten Aminoalkohole der Formel (IV) sind gemäß EP 0 143 306 A2 ausgehend von den entsprechenden Mannich-Basen (III) durch Reduktion mit einem Reduktionsmittel, wie z.B. Natriumborhydrid, Natriumcyanoborhydrid, Lithiumaluminiumhydrid, Düsobutylaluminiumhydrid oder einem komplexen Analogon dieser Verbindungen, bei -70 bis +110 °C in geeigneten Lösungsmitteln, z.B. Diethylether, THF, Methanol oder Ethanol, erhältlich. Geht man beispielsweise von einer Mannich-Base mit anti-Konfiguration (anti-III) aus, so wird bei Reduktion mit $NaBH_4$ in Ethanol bei Raumtemperatur und einer Reaktionszeit von 8 bis 16h der entsprechende (anti, anti-IV) Aminoalkohol erhalten. Wird hingegen zur Reduktion der Mannich-Base (anti-III) DIBAH oder L-Selectride in THF verwendet, so erhält man den (syn,anti-IV)-Aminoalkohol ih-ticiher-Diastereomerenreinheit. Bei Reduktion einer Mannich-Base (III), die nicht in diastereomerenreiner oder -angereicherter Form vorliegt, wird üblicherweise eine Mischung der verschiedenen Stereoisomeren des Aminoalkohols (IV) erhalten, die - falls erforderlich - mit bekannten Methoden (Kristalisation, Chromatographie) in die Diastereomeren und ggf. auch die Enantiomeren aufgetrennt werden kann.

Alternativ zu dem Tosyl/Mesyl-Azid-Verfahren kann der Aminoalkohol (IV) auch mittels Mitsunobu-Reaktion durch Umsetzung zunächst mit Azodicarbonsäuredimethyl- oder -diethylester, Triphenylphosphan und einem Phthalimid und anschließend mit Hydrazin in das korresponidierende Diamin (1) überführt werden (O. Mitsunobu, Synthesis (1981) 1-28). Da diese Reaktion unter Inversion der Stereochemie am O-Kohlenstoffatom abläuft, kann mit ihrer Hilfe aus dem Alkohol (anti,anti-IV) stereoselektiv das Diamin (syn,anti-I), aus (syn,anti-IV) hingegen stereoselektiv das Diamin (anti, anti-I) erhalten werden.

In einem weiteren erfindungsgemäßen Verfahren werden Verbindungen der allgemeinen Struktur (I) mit $R^3$ = H, n-Propyl oder -$CH_2$-Phenyl und $R^4$ H - und zwar bevorzugt die Diasteromeren (syn,anti-I) (mit der relativen Konfiguration syn,anti) erhalten -, das durch folgende Verfahrensschritte gekennzeichnet ist:

(aa) Umsetzung eines Imins der allgemeinen Struktur (VII)

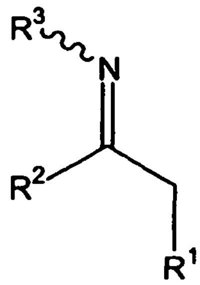

VII ,

worin $R^1$ und $R^2$ wie für Formel (I) definiert sind und $R^3$ H, n-Propyl oder -$CH_2$-Phenyl bedeutet, mit einem Iminiumsalz der allgemeinen Struktur (VIII)

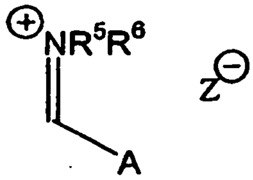

VIII ,

worin $R^5$, $R^6$, A und $Z^\ominus$ wie oben definiert sind; und

(bb) anschließende Reduktion des oder der in dem Verfahrensschritt (aa) gebildeten Zwischenprodukts/e. Vorzugsweise erfolgt die Reduktion mit einem komplexen Hydrid oder mit molekularem Wasserstoff ($H_2$-Partialdruck von 1 bis 50 bar) in Gegenwart eines Übergangsmetall-Katalysators (Ni, Pd, Pt, $PtO_2$).

Geeignete komplexe Hydride sind z.B. Natriumborhydrid, Natriumcyanoborhydrid, Lithiumaluminiumhydrid, Düsobutytaluminiumhydrid oder ein komplexes Analogon dieser Verbindungen, die bei -70 bis +110 ˚C in geeigneten Lösungsmitteln, z.B. Diethylether, THF, Methanol oder Ethanol, ggf. im Gemisch mit Methylenchlorid, eingesetzt werden können.

Die Imine (VII) sind ausgehend von den entsprechenden Ketonen (IX) durch Umsetzung mit Ammoniak oder Ammoniumacetat ($R^3$ = H) bzw primären Aminen $R^3NH_2$ ($R^3 \neq$ H) nach literaturbekannten Verfahren erhältlich (J. March, Advanced Organic Chemistry, New York, Chichester, Brisbane, Toronto, Singapore, 3. Aufl., (1985), S. 796-798).

Wird bei diesem (Imin+Iminiumsalz)-Verfahren ein Imin (VII) verwendet, für das $R^3$ -($CH_2$)-Phenyl bedeutet, handelt es sich also bei dem Imin (VII) um ein N-Benzyl-substitulertes Imin, so kann dieser Benzylrest im erfindungsgemäßen Produkt (I) mit $R^3$ = Benzyl durch Umsetzung mit Wasserstoff ($H_2$) in Gegenwart eines Übergangmetalls (z.B. Palladium, Platin oder Nickel) entfernt und so das Diamin (I) mit $R^3$ = $R^4$ = H erhalten werden. Dieser Verfahrensschritt (cc) wird vorzugsweise mit 10% Palladium an Kohlenstoff als Übergangmetall, bevorzugt in Methanol, ausgeführt.

Mit diesem erfindungsgemäßen Verfahren sind somit auch syn,anti-konfigurierte Diamine der allgemeinen Struktur (I) diastereoselektiv zugänglich.

Verbindungen der allgemeinen Struktur (I) mit $R^3$ = H und $R^4$ = H können - unabhängig davon, ob sie als Racemat, in Form eines oder mehrerer Diastereomeren oder eines oder mehrerer Enantiomeren vorliegen - durch Umsetzung mit einem Acylierungsreagenz in die korrespondierenden Verbindungen der allgemeinen Struktur (I) mit $R^3$ = C(=O)-$R^7$ überführt werden, wobei $R^7$ wie oben definiert ist. Vorzugsweise ist das Acylierungsmittel ein Säurechlorid der allgemeinen Formel $R^7$-C(=O)-Cl, worin $R^7$ Methyl, Phenyl, 2-Fluorphenyl, 2-Chlorphenyl oder 2-Methylphenyl bedeutet.

In literaturbekannter Weise können die Verbindungen der allgemeinen Struktur (I) mit $R^3$ = H und $R^4$ = H auch alkyliert oder einer reduktiven Aminierung mit Aldehyden oder Ketonen (s. z.B. J. March, Advanced Organic Chemistry, New York, Chichester, Brisbane, Toronto, Singapore, 3. Aufl., (1985), 798-800) unterworfen werden, so daß die entsprechenden Verbindungen (I), in denen $R^3$ n-Propyl oder -$CH_2$-Phenyl und $R^4$ H bedeutet, ohne weiteres zugänglich sind. Diamine der allgemeinen Struktur (I) mit $R^3$ = H können dann ebenfalls einer Acylierung (so daß $R^3$ -C(=O)-$R^7$

bedeutet) unterworfen werden, vorzugsweise mit einem wie oben definierten Säurechlorid Cl-C(=O)-$R^7$.

Die erfindungsgemäßen Verbindungen der allgemeinen Formel (I), in denen der Rest $R^3$ gleich n-Propyl oder -$CH_2$-Phenyl ist und $R^4$ = H bedeutet, sind beispielsweise auch durch Umsetzung des entsprechenden Imins, das in Form seines tautomeren Enamins (XII) vorliegt, mit einem entsprechenden Iminiumsalz (VIII) und anschließender Reduktion mit beispielsweise $NaBH_4$ in Methanol zugänglich (Synlett (1997) 177-178).

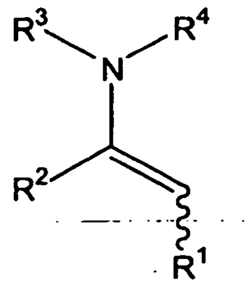

XII

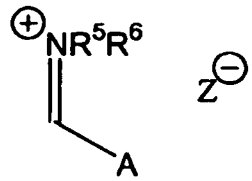

VIII

Dabei entsteht bevorzugt das syn,anti-Diastereomere der Verbindung (I).

Die in den zur Herstellung der erfindungsgemäßen Diamine der allgemeinen Struktur (I) verwendeten Verfahren eingesetzten Ausgangsverbindungen, Reagenzien und Lösungsmittel sind, soweit in der Beschreibung nichts anderes angegeben ist, kommerziell erhältlich (von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan) oder können nach im Stand der Technik allgemein bekannten Verfahren hergestellt werden.

Die erfindungsgemäßen Verbindungen der allgemeinen Struktur (I) können sowohl in Substanz als auch als Salz isoliert werden. Die erfindungsgemäße Verbindung der allgemeinen Struktur (I) wird üblicherweise nach erfolgter Umsetzung gemäß dem oben beschriebenen erfindungsgemäßen Verfahren und anschließender herkömmlicher Aufarbeitung erhalten. Die so gewonnene oder in-situ ohne Isolierung gebildete Verbindung der allgemeinen Struktur (I) kann dann beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure. Citronensäure, Glutaminsäure oder Aspaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Die besonders bevorzugte Hydrochloridbildung kann auch durch Versetzen der in einem geeigneten organischen Lösungsmittel, wie z.B. Butan-2-on (Methylethylketon), gelösten Base mit Trimethylsilylchlorid (TMSCl), vorteilhaft in Gegenwart von Wasser, herbeigeführt werden.

Soweit die Verbindungen der allgemeinen Struktur (I) in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u. a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, gebildeten diastereomeren Salzen voneinander getrennt werden.

Ein weiterer Gegenstand der vorliegenden Erfindung ist ein Arzneimittel, enthaltend mindestens eine wie oben definierte Verbindung der allgemeinen Struktur (I), oder eines ihrer pharmazeutischen Salze, insbesondere das Hydrochlorid-Salz. Vorzugsweise enthält das erfindungsgemäße Arzneimittel in einer pharmazeutischen Zusammensetzung mindestens eine der oben beispielhaft genannten Verbindungen in Substanz oder als pharmazeutisch annehmbares Salz und gegebenenfalls weitere Wirk- und Hilfsstoffe. Dabei kann das erfindungsgemäße Diamin (I) als Racemat, in Form eines oder mehrerer Diastereomeren oder eines oder mehrerer Enantiomeren vorliegen.

Da sich die erfindungsgemäßen Verbindungen der allgemeinen Struktur (I) überraschend als analgetisch wirksam erwiesen haben, werden die sie enthaltenden erfindungsgemäßen Arzneimittel vorzugsweise in der Prophylaxe und/oder Therapie von Schmerzzuständen, wie z. B. akuter Schmerz, chronischer Schmerz bzw. neuropathischer Schmerz, insbesondere starker bis stärkster Schmerzen, eingesetzt. Es hat sich auch gezeigt, daß die erfindungsgemäßen Arzneimittel zur Behandlung und/oder Prophylaxe von Diarrhoe, Harninkontinenz, Juckreiz und/oder Tinnitus aurium eingesetzt werden können.

Ein weiterer Gegenstand der vorliegenden Erfindung betrifft die Verwendung eines Diamins der Formel (I) oder

eines seiner pharmazeutisch annehmbaren Salze zur Herstellung eines Medikaments zur Prophylaxe und/oder Behandlung von Schmerz, Diarrhoe, Harninkontinenz, Juckreiz und/oder Tinnitus aurium.

Die erfindungsgemäßen Arzneimittel, Medikamente und pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, transdermale therapeutische Systeme, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Struktur (I) je nach galenischer Form und in Abhängigkeit vom Applikationsweg pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kökusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel/Medikament oral, subkutan, parenteral, intravenös, vaginal, pulmonal, intraperitoneal, transdermal, intramuskulär, nasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Pulver zur Inhalation sowie Sprays. Erfindungsgemäße Verbindungen der allgemeinen Struktur (I) in einem Depot in gelöster Form oder in einem Pflaster, gegebenenfalls unter Zusatz von die Hautpenetration fördernden Mitteln, sind geeignete perkutane Applikationszubereitungen. Rektal, transmucosal, parenteral, oral oder perkutan anwendbare Zubereitungsformen können die erfindungsgemäßen Verbindungen der allgemeinen Struktur (I) verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur (I) oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u. a. polymere Säuren und Mischungen von polymeren Säuren mit Materialien wie z.B. Schellack, Cetylalkohol und/oder Celluloseacetat. Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,005 bis 500 mg/kg, insbesondere 0,05 bis 5 mg/kg, vorzugsweise 2 bis 250 mg/kg Körpergewicht wenigstens einer erfindungsgemäßen Verbindung der allgemeinen Struktur (I) appliziert.

Nachfolgend wird die vorliegende Erfindung durch Beispiele weiter erläutert, ohne sie darauf zu beschränken.

**Beispiele**

Vorbemerkungen

Die eingesetzten Chemikalien und Lösungsmittel wurden von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen bzw.

TCI, Japan durch Kauf erworben oder nach üblichen und im Stand der Technik bekannten Verfahren synthetisiert.

Wasserfreies THF wurde frisch über Kalium unter einer Argon-Atmosphäre destilliert.

Dünnschichtchromatographische Untersuchungen wurden mit HPTLC-Fertigplatten, Kieselgel 60 F 254, der Firma E. Merck, Darmstadt, durchgeführt. Als stationäre Phase für die Säulenchromatographie und MPLC wurde Kieselgel 60 (0.040 - 0.063 mm) der Firma E. Merck, Darmstadt, oder $Al_2O_3$, neutral, der Firma Macherey-Nagel, Düren, eingesetzt.

Die Ausbeuten der hergestellten Verbindungen sind nicht optimiert. Alle angegebenen Temperaturen sind unkorrigiert.

Die Mischungsverhältnisse der Laufmittel für chromatographische Untersuchungen sind stets in Volumen/Volumen (V/V) angegeben.

ESI-Massenspektren wurden aufgenommen mit einem LCQ Classic Massenspektrometer der Firma Finnigan, die $^1$H- und $^{13}$C-NMR-Spektren wurden mit einem 300-(75-)MHz- Avance-DPX-300-NMR-Gerät, einem 600-(150-)MHz-Avance-DRX-600-NMR-Gerät oder einem Bruker-ARX-200-NMR-Gerät der Firma Bruker aufgenommen, wobei Tetramethylsilan als interner Standard verwendet wurde. IR-Spektren wurden mit einem Nicolet 510 P FT-IR Spektrometer aufgenommen. GC/MS-Daten wurden mit einem Finnigan MAT Magnum System 240-Gerät erhalten. Elementaranatysen , wurden, soweit durchgeführt, mit einem Perkin Elmer Elemental Analyser durchgeführt und lieferten hinreichende Elementaranalysen-Ergebnisse: C $\pm$ 0.34, H $\pm$ 0.28, N $\pm$ 0.19.

<u>Allgemeine Arbeitsvorschrift 1 (AAV 1: Imin+Iminiumsalz-Verfahren)</u>

Die Reaktionen wurden unter einer Argon-Atmosphäre ausgeführt. Eine Lösung des Imins (VII) (2,5 mmol) in wasserfreiem $CH_2Cl_2$ (2,5 mL) wurde auf -80 ˚C abgekühlt. Das Iminiumsalz (VIII) (2,5 mmol) wurde dann unter Rühren in einer Portion hinzugegeben. Die Mischung wurde gerührt, und man ließ die Temperatur über 2-3 h auf -30 ˚C ansteigen. Die Reaktionsmischung wurde für 15 h bei dieser Temperatur in einem Tiefkühlgerät aufbewahrt. Dann wurde $NaBH_4$ (40 mmol) in MeOH (10 mL) hinzugegeben, und man ließ die Temperatur auf Raumtemperatur ansteigen. Nach 5-stündigem Rühren bei Umgebungstemperatur wurde HCl (5 mL, 6 N) hinzugegeben, und die Mischung wurde einige Male mit $Et_2O$ gewaschen. Anschließend wurden die wäßrige Schicht durch Zugabe von $NH_3$ (25 % $NH_3$: $H_2O$ =1:1) alkalisch gemacht und das erfindungsgemäße Diamin (I) mit $CH_2Cl_2$ (3 x 50 mL) extrahiert. Die vereinigten organischen Phasen wurden über $Na_2SO_4$ getrocknet. Das Lösungsmittel wurde an einem Rotationsverdampfer entfernt, und der Rückstand wurde mittels Säulenchromatographie an $Al_2O_3$ ($CH_2Cl_2$ /MeOH) gereinigt. Die letzte eluierte Fraktion war das Diamin (I).

<u>Allgemeine Arbeitsvorschrift 2 (AAV 2: Debenzylierung des Diamins (I) mit $R^3$ = $-CH_2$-Phenyl)</u>

Eine Lösung des benzylierten Diamins (I) in wasserfreiem MeOH (10 mL) wurde bei Raumtemperatur in Gegenwart von 10 % Pd / C(20 mg) gerührt, und $H_2$ wurde in die Mischung eingeleitet, bis die Debenzylierung vollständig war (DC-Kontrolle). Nach Entfernen des Katalysators mittels Filtration über Celite wurde das Filtrat eingedampft, um das debenzylierte Diamin (I) zu ergeben. Der Rückstand wurde mittels Säulenchromatographie an $Al_2O_3$ ($CH_2Cl_2$ / MeOH = 95 : 5) gereinigt.

<u>Allgemeine Arbeitsvorschrift 3 (AAV 3: Azid-Methode)</u>

<u>Herstellung der Mannich-Basen (III)</u>

Zu einer Lösung von wasserfreiem NaI (getrocknet bei 140˚C im Vakuum) in trockenem MeCN (5,5 mmol; c $\approx$ 1 mol/l) wurden Dimethylamin-Hydrochlorid (2,5 mmol), $NEt_3$ (5 mmol) und $Me_3SiCl$ (5,5 mmol) hinzugegeben. Nach Rühren für 30 min bei Umgebungstemperatur wurde der Aldehyd A-CHO (2,5 mmol) hinzugegeben, und das Rühren wurde für weitere 30 min fortgesetzt. Dann wurde als Enamin 1-(Pyrrolidino)-1-cyclohexen (2,5 mmol) hinzugegeben, und die Mischung wurde für weitere 60 min gerührt. Danach wurde die Mischung mit wäßr. HCl (5 mL, 37 % HCl : $H_2O$ = 1 : 1) angesäuert, für 10 min gerührt und mit $Et_2O$ (3 x 50 mL) gewaschen. Anschließend wurde verdünnter $NH_3$ (25 mL, 25 % $NH_3$ : $H_2O$ = 1 : 4) unter heftigem Rühren hinzugegeben, und die Mannich-Base (III) wurde mit $CH_2Cl_2$ oder $Et_2O$ (3 x 50 mL) extrahiert. Die vereinigten organischen Phasen wurden über $Na_2SO_4$ getrocknet. Das Lösungsmittel 2 wurde schließlich an einem Rotationsverdampfer ohne Erwärmen entfernt.

<u>Herstellung der Aminoalkohole (IV)</u>

Die Mannich-Base (III) (1 mmol) wurde in Ethanol 10 mL) gelöst, $NaBH_4$ (2,5 mmol) wurde hinzugegeben, und die Mischung wurde für 5 h bei Raumtemperatur gerührt. Anschließend wurde wäßr. HCl (37 % HCl : $H_2O$ = 1 : 1, 10

mL) hinzugegeben, und die Mischung wurde einige Male mit $Et_2O$ (50 mL) gewaschen. Die wäßrige Schicht wurde durch Zugabe von $NH_3$ (25 % $NH_3$ : $H_2O$ = 1 : 1) alkalisch gemacht. Das Produkt wurde mit $CH_2Cl_2$ (3 x 50 mL) extrahiert, und die organische Phase wurde über $Na_2SO_4$ getrocknet. Das Lösungsmittel wurde im Vakuum entfernt, um ein gelbes Öl zu erhalten. Das Produkt (IV) wurde ohne weitere Reinigung verwendet.

Mesylierung des Aminoalkohols (IV)

Zu einer Lösung des Aminoalkohols (IV) (2 mmol) in $CH_2Cl_2$ (5 mL) wurden bei 0˚C Mesylchlorid (2,4 mmol) und $NEt_3$ (3 mmol) hinzugegeben. Nach 1 h war die Reaktion vollständig (DC-Kontrolle). Die Mischung wurde mit $CH_2Cl_2$ (10 mL) verdünnt und zweimal mit wäßr. $Na_2CO_3$-Lösung und einmal mit Salzlösung gewaschen. Die organische Phase wurde mit $Na_2SO_4$ getrocknet, um das Mesylat (V) als ein gelbes Öl zu ergeben, das in den nachfolgenden Reaktionen ohne weitere Reinigung eingesetzt wurde.

Bildung des Azids (VI)

Eine Lösung von $NaN_3$ (20 mmol) und des Mesylats (V) (2 mmol) in DMSO (40 mL) wurde für 3 h auf 50˚C erhitzt. Das DC zeigte den vollständigen Verbrauch des Ausgangsmaterials. Die Reaktion wurde mit Salzlösung gequencht und mit $CH_2Cl_2$ (50 mL) extrahiert. Die organische Phase wurde dreimal mit gesättigter $Na_2CO_3$-Lösung und einmal mit Salzlösung gewaschen. Nach dem Trocknen über $Na_2SO_4$ wurde das Azid (VI) als ein braunes Öl erhalten. Das rohe Produkt (VI) wurde ohne weitere Reinigung in der folgenden Reaktion eingesetzt.

Reduktion des Azids (VI) zum Diamin (I)

Eine Lösung des Azids (VI) (1 mmol) in $Et_2O$ wurde langsam zu einer Suspension von $LiAlH_4$ (1,5 mmol) in $Et_2O$ hinzugegeben. Nach 4 h wurde die Reaktion sehr langsam mit Wasser und HCl (37 % HCl: $H_2O$ = 1 : 1) gequencht. Nach dem Alkalischmachen wurde das Produkt mit $Et_2O$ (3 x 50 mL) extrahiert und mit Wasser (50 mL) gewaschen. Die organische Phase wurde mit $Na_2SO_4$ getrocknet und an $Al_2O_3$ ($CH_2Cl_2$ / MeOH = 95 : 5) chromatographiert, um das Diamin (I) als ein gelbliches Öl zu ergeben.

Allgemeine Arbeitsvorschrift 4 (AAV 4; Aminoimin(II)-Reduktions-Verfahren) Variante (A)

Eine Lösung von Ammoniumacetat (12,1 mmol) und der Mannich-Base (III) (1,8 mmol) in THF wurde für 1 h bei Raumtemperatur gerührt. Eine Lösung von L-Selectride in THF (3,6 mmol) wurde bei 0˚C hinzugegeben, man ließ die Temperatur auf Raumtemperatur ansteigen und rührte weiter über Nacht. HCl (5 mL, 6 N) wurde hinzugegeben und die Mischung einige Male mit $Et_2O$ gewaschen. Anschließend wurde die wäßrige Phase mit $NH_3$ (25 % $NH_3$ : $H_2O$ = 1 : 1) alkalisch gemacht, und das Diamin (I)wurde mit $CH_2Cl_2$ (3 x 50 mL) extrahiert. Die vereinigten organischen Phasen wurden über $Na_2SO_4$ getrocknet. Das Lösungsmittel wurde an einem Rotationsverdampfer entfernt, und der Rückstand wurde mittels Säulenchromatographie an $Al_2O_3$ ($CH_2Cl_2$ / MeOH) gereinigt. Die letzte eluierte Fraktion war das Diamin (I).

Variante B

Eine Lösung von Ammoniumacetat (12,1 mmol) und der Mannich-Base (III) (1,8 mmol) in THF wurde für 1 h bei Raumtemperatur gerührt. Eine Lösung von DIBAH in n-Hexan (3,6 mmol) wurde bei 0˚C hinzugegeben. Man ließ die Temperatur auf Raumtemperatur ansteigen und rührte weiter über Nacht. HCl (5 mL, 6 N) wurde hinzugegeben und die Mischung einige Male mit $Et_2O$ gewaschen. Anschließend wurden die wäßr. Phase durch Zugabe von $NH_3$ (25% $NH_3$:$H_2O$ = 1 : 1) alkalisch gemacht und das Diamin (I) mit $CH_2Cl_2$ (3 x 50 ml) extrahiert. Die vereinigten organischen Phasen wurden über $Na_2SO_4$ getrocknet. Das Lösungsmittel wurde an einem Rotationsverdampfer entfernt und der Rückstand mittels Säulenchromatographie an $Al_2O_3$ ($CH_2Cl_2$ / MeOH) gereinigt. Die letzte eluierte Fraktion war das Diamin (I).

Variante C

$NaCNBH_3$ (2,1 mmol) wurde zu einer Suspension von $ZnCl_2$ in MeOH bei 0˚C hinzugegeben. Nach Rühren für 1 h bei dieser Temperatur wurden die Männich-Base (III) (1,8 mmol) und Ammoniumacetat (12,1 mmol) in einer Portion hinzugegeben. Die Mischung wurde gerührt und man ließ die Temperatur auf Raumtemperatur ansteigen. Es wurde weiter über Nacht gerührt. HCl (5 mL, 6 N) wurde hinzugegeben und die Mischung einige Male mit $Et_2O$ gewaschen. Anschließend wurden die wäßrige Phase durch Zugabe von $NH_3$ (25 % $NH_3$ : $H_2O$ = 1 : 1) alkalisch gemacht und das Diamin (I) mit $CH_2Cl_2$ (3 x 50 mL) extrahiert. Die vereinigten organischen Phasen wurden über $Na_2SO_4$ getrocknet. Das

Lösungsmittel wurde an einem Rotationsverdampfer entfernt, und der Rückstand wurde mittels Säulenchromatographie an $Al_2O_3$ ($CH_2Cl_2$ / MeOH) gereinigt. Die letzte eluierte Fraktion war das Diamin (I).

Allgemeine Arbeitsvorschrift 5 (AAV 5; Acylierungsverfahren)

Das Reaktionsgefäß wurde im Trockenschrank ausgeheizt. Das Diamin (I) (mit $R^3 = R^4 = H$) (600 mg) wurde vorgelegt, und eine Lösung von 1,3 Moläquivalenten Triethylamin in Dichlormethan (V/V = 1 : 8), die eine Spur 4-Dimethylaminopyridin enthielt, wurde zugegeben. Anschließend wurden 1,3 Moläquivalente des Säurechlorids $R^7$-C(=O)-Cl bei -10 °C zugegeben und über Nacht unter Erwärmung auf Raumtemperatur gerührt. Nach erneuter Abkühlung bis -10°C wurden 2 ml 5 N KOH-Losung zugegeben, die Phasen getrennt und die organische Phase nochmals mit 4 ml 0,1 N KOH-Lösung gewaschen. Die organische Phase wurde über Magnesiumsulfat getrocknet und bei 40 °C im Vakuum eingeengt. Das erhaltene Rohprodukt wurde über MPLC (Fließmittel n-Hexan; allmählicher Zusatz von Diethylether auf bis zu 100%) aufgereinigt. Die abschließende Hydrochloridfällung erfolgte durch Lösen der Rohbase in ca. 10 ml 2-Butanon je Gramm Base, anschließende Zugabe eines halben Moläquivalents Wasser, gefolgt von 1,1 Moläquivalenten Chlortrimethylsilan und Rühren über Nacht. Das ausgefallene Hydrochlorid wurde abfiltriert und im Vakuum getrocknet.

Allgemeine Arbeitsvorschrift 6 (AAV 6; Hydrochlorid-Bildung)

Zur Hydrochlorid-Fällung wurde die Rohbase (I) in ca. 10 ml 2-Butanon je Gramm Base aufgenommen. Dann wurden 0,5 Moläquivalente Wasser, gefolgt von 1,1 Moläquivalenten Chlortrimethylsilan hinzugegeben und über Nacht gerührt. Das ausgefallene Hydrochlorid wurde abfiltriert und im Vakuum getrocknet.

Die nach den AAV 1-6 beispielhaft hergestellten Verbindungen sind in Tabelle 1 wiedergegeben. Die Bestimmung der Stereochemie erfolgte mittels $^1H$- und $^{13}C$-NMR-Untersuchungen, insbesondere durch Vergleich der chemischen Verschiebungen der C-Atome C-$NR^3R^4$, C-$R^1$ und C-A im $^{13}C$-NMR-Spektrum der erfindungsgemäßen Verbindungen untereinander und mit den Verschiebungen der entsprechenden C-Atome im $^{13}C$-NMR-Spektrum von (anti, anti)-1-Hydroxy-2-(pyrrolidin-phenyl-methyl)-cyclohexanund(syn,anti)-1-Hydroxy-2-(pyrrolidin-phenyl-methyl)-cyclohexan.

Tabelle 1

| Beispiels-Nr. | Verbindung | Herstellungsverfahren (AAV) |
|---|---|---|
| 1 | (syn,syn)-N-[2-(Dimethylaminopyridin-3-ylmethyl)cyclohexyl] benzamid-Hydrochlorid | 4A/B + 5 + 6 |
| 1a | (syn,syn)-2-(Dimethylaminopyridin-3-ylmethyl)cyclohexylamin | 4A/B |
| 2 | (syn,syn)-N-[2-(Dimethylaminopyridin-3-ylmethyl)cyclohexyl]-2-fluorbenzamid-Hydrochlorid | 4A/B + 5 + 6 |
| 3 | (syn,syn)-2-Chlor-N-[2-(Dimethylaminopyridin-3-ylmethyl) cyclohexyl]-benzamid-Hydrochlorid | 4A/B + 5 + 6 |
| 4 | (syn,syn)-N-[2-(Dimethylaminopyridin-3-ylmethyl)cyclohexyl]-2-methylbenzamid-Hydrochlorid | 4A/B + 5 + 6 |
| 5 | (anti,anti)-N-[2-(Dimethylaminopyridin-3-ylmethyl) cyclohexyl]-benzamid-Hydrochlorid | 4C + 5 + 6 |
| 5a | (anti,anti)-2-(Dimethylaminopyridin-3-ylmethyl)cyclohexylamin | 4C |
| 6 | (anti,anti)-N-[2-(Dimethylaminopyridin-3-ylmethyl)cyclo-hexyl]-2-fluorbenzamid-Hydrochlorid | 4C + 5 + 6 |
| 7 | (anti,anti)-2-Chlor-N-[2-(dimethylaminopyridin-3-ylmethyl) cyclohexyl]benzamid-Hydrochlorid | 4C + 5 + 6 |
| 8 | (anti,anti)-N-[2-(Dimethylaminopyridin-3-ylmethyl)cyclohexyl]-2-methylbenzamid-Hydrochlorid | 4C + 5 + 6 |
| 9 | (syn,syn)-N-[2-(Dimethylaminopyridin-3-ylmethyl)cyclohexyl] acetamid-Hydrochlorid | 4A/B + 5 + 6 |
| 10 | (anti,anti)-N-[2-(Dimethylaminopyridin-3-ylmethyl)cyclohexyl] acetamid-Hydrochlorid | 4C + 5 + 6 |

(fortgesetzt)

| Beispiels-Nr. | Verbindung | Herstellungsverfahren (AAV) |
|---|---|---|
| 11 | (syn,syn)-N-[2-(Dimethylaminophenylmethyl)cyclohexyl]-2-fluorbenzamid-Hydrochlorid | 4A/B + 5 + 6 |
| 11a | (syn,syn)-2-(Dimethylaminophenylmethyl)cyclohexylamin | 4A/B |
| 12 | (syn,syn)-N-[2-(Dimethylamino-phenyl-methyl)-cyclohexyl]-acetamid-Hydrochlorid | 4A/B+5+6 |
| 13 | (syn,syn)-N-[2-(Dimethylamino-phenyl-methyl)-cyclohexyl]-benzamid-Hydrochlorid | 4A/B+5+6 |
| 14 | (syn,syn)-2-Chlor-N-[2-(dimethylamino-phenyl-methyl)-cyclohexyl]-benzamid-Hydrochlorid | 4A/B + 5 + 6 |
| 15 | (syn,syn)-N-[2-(Dimethylamino-phenyl-methyl)-cyclohexyl]-2-methyl-benzamid-Hydrochlorid | 4A/B + 5 + 6 |
| 16 | (anti,anti)-N-[2-(Dimethylamino-phenyl-methyl)-cyclohexyl]-acetamid-Hydrochlorid | 4C + 5 + 6 |
| 16a | (anti,anti)-2-(Dimethylamino-phenyl-methyl)-cyclohexylamin | 4C |
| 17 | (anti,anti)-N-[2-(Dimethylamino-phenyl-methyl)-cyclohexyl]-benzamid-Hydrochlorid | 4C + 5 + 6 |
| 18 | (anti,anti)-N-[2-(Dimethylamino-phenyl-methyl)-cyclohexyl]-2-methyl-benzamid-Hydrochlorid | 4C + 5 + 6 |
| 19 | (syn,syn)-2-Chlor-N-{2-[(2-chlor-phenyl)-dimethylamino-methyl]-cyclohexyl}-benzamid-Hydrochlorid | 4A/B + 5 + 6 |
| 19a | (syn,syn)-2-[(2-Chlor-phenyl)-dimethylamino-methyl]-cyclohexylamin | 4A/B |
| 20 | (anti,anti)-2-Chlor-N-{2-[(2-chlor-phenyl)-dimethylamino-methyl]-cyclohexyl}-benzamid-Hydrochlorid | 4C + 5 + 6 |
| 20a | (anti,anti)-2-[(2-Chlor-phenyl)-dimethylamino-methyl]-cyclohexylamin | 4C |
| 21 | (syn,syn)-N-{2-[(2-Chlor-phenyl)-dimethylamino-methyl]-cyclohexyl}-2-fluor-benzamid-Hydrochlorid | 4A/B + 5 + 6 |
| 22 | (anti,anti)-N-{2-[Dimethylamino-(2-nitro-phenyl)-methyl]-cyclohexyl}-benzamid-Hydrochlorid | 4C + 5 + 6 |
| 22a | (anti,anti)-2-[Dimethylamino-(2-nitro-phenyl)-methyl]-cyclohexylamin | 4C |
| 23 | (anti,anti)-N-{2-[Dimethylamino-(2-nitro-phenyl)-methyl]-cyclohexyl}-2-fluor-benzamid-Hydrochlorid | 4C + 5 + 6 |
| 24 | (anti,anti)-2-Chlor-N-{2-[dimethylamino-(2-nitro-phenyl)-methyl]-cyclohexyl}-benzamid-Hydrochlorid | 4C + 5 + 6 |
| 25 | (anti,anti)-N-{2-[Dimethylamino-(2-nitro-phenyl)-methyl]-cyclohexyl}-2-methyl-benzamid-Hydrochlorid | 4C + 5 + 6 |
| 26 | (syn,syn)-N-{2-[Dimethylamino-(2-nitro-phenyl)-methyl]-cyclohexyl}-acetamid-Hydrochlorid | 4A/B + 5 + 6 |
| 26a | (syn,syn)-N-2-[Dimethylamino-(2-nitro-phenyl)-methyl]-cyclohexylamin | 4A/B |
| 27 | (anti,anti)-N-{2-[(2-Chlor-phenyl)-dimethylamino-methyl]-cyclohexyl}-acetamid-Hydrochlorid | 4C + 5 + 6 |

(fortgesetzt)

| Beispiels-Nr. | Verbindung | Herstellungsverfahren (AAV) |
|---|---|---|
| 28 | (syn,anti)-2-(Dimethylamino-phenyl-methyl)-cyclohexylamin | 1 + 2 |
| 29 | (syn,anti)-N-[2-(Dimethylamino-phenyl-methyl)-cyclohexyl]-benzamid | 1 |
| 30 | (anti,anti)-N-{2-[Dimethylamino-(2-methoxy-phenyl)-methyl]-cyclohexyl}-benzamid | 4C + 5 |
| 30a | (anti,anti)-2-[Dimethylamino-(2-methoxy-phenyl)-methyl]-cyclohexylamin | 4C |
| 31 | (anti,anti)-N-{2-[Dimethylamino-(2-nitro-phenyl)-methyl]-cyclohexyl}-benzamid | 4C + 5 |
| 33 | (anti,anti)-N-{2-[(2-Chlor-phenyl)-dimethylamino-methyl]-cyclohexyl}-benzamid | 4C + 5 |
| 35 | (anti,anti)-N-{2-[Dimethylamino-(2-methoxy-phenyl)-methyl]-cyclohexyl}-acetamid | 4C + 5 |
| 36 | (anti,anti)-{2-[(2-Chlor-phenyl)-dimethylamino-methyl]-cyclohexyl}-acetamid | 4C + 5 |
| 37 | (anti,anti)-N-{2-[Dimethylamino-(2-nitro-phenyl)-methyl]-cyclohexyl}-acetamid | 4C + 5 |
| 38 | (syn,syn)-2-(Dimethylamino-phenyl-methyl)-cyclohexylamin | 4A/B |
| 40 | (anti,anti)-2-Chlor-N-(3-dimethylamino-1-ethyl-2-methyl-3-phenyl-propyl)-benzamid | 4C + 5 |
| 40a | (anti,anti)-3-Dimethylamino-1 -ethyl-2-methyl-3-phenyl-propylamin | 4C |
| 41 | (syn,anti)-2-(Dimethylamino-phenyl-methyl)-cyclohexyl-N-(n-propyl)-amin | 1 |
| 42 | (syn,anti)-2-(Morpholin-4-yl-phenyl-methyl)-cyclohexyl-N-(n-propyl)-amin | 1 |
| 43 | (syn,anti)-2,N,N-Trimethyl-1,3-diphenyl-N'-propy-propan-1,3-diamin | 1 |
| 44 | (syn,anti)-2-(Dimethylamino-phenyl-methyl)-cyclohexyl-N-benzyl-amin | 1 |
| 45 | (syn,anti)-2-(Morpholin-4-yl-phenyl-methyl)-cyclohexyl-N-benzyl-amin | 1 |
| 46 | (syn,anti)-2,N,N-Trimethyl-1,3-diphenyl-N'-benzyl-propan-1,3-diamin | 1 |
| 47 | (syn,anti)-2-(Dimethylamino-phenyl-methyl)-cyclohexylamin | 1 + 2; 3 |
| 48 | (syn,anti)-2-(Morpholin-4-yl-phenyl-methyl)-cyclohexylamin | 1+2 |
| 49 | (syn,anti)-2,N,N-Trimethyl-1,3-diphenyl-propan-1,3-diamin | 1+2 |
| 50 | (syn,anti)-2-[(2-Chlorphenyl)-dimethylamino-methyl]-cyclohexylamin | 3 |
| 51 | (anti,anti)-2-[(2-Chlorphenyl)-dimethylamino-methyl]-cyclohexylamin | 4C |
| 52 | (syn,syn)-2-(Dimethylamino-phenyl-methyl)-cyclohexylamin | 4A/B |
| 53 | (anti,anti)-2-(Dimethylamino-phenyl-methyl)-cyclohexylamin | 4C |

(fortgesetzt)

| Beispiels-Nr. | Verbindung | Herstellungsverfahren (AAV) |
|---|---|---|
| 54 | (syn,syn)-2-[(2-Chtorphenyl)-dimethylamino-methyl]-cyclohexylamin | 4A/B |
| 55 | (syn,syn)-2-(Dimethylamino-pyridin-3-yl-methyl)-cyclohexylamin | 4A/B |
| 56 | (anti,anti)-2-(Dimethylamino-pyridin-3-yl-methyl)-cyclohexylamin | 4C |
| 57 | (syn,syn)-2-(Dimethylamino-(2-methoxyphenyl)-methyl)-cyclohexylamin | 4A/B |
| 58 | (ant,anti)-2-(Dimethylamino-(2-methoxyphenyl)-methyl)-cyclohexylamin | 4C |
| 59 | (syn,syn)-2-(Dimethylamino-(2-nitrophenyl)-methyl)-cyclohexylamin | 4A/B |
| 60 | (anti,anti)-2-(Dimethylamino-(2-nitrophenyl)-methyl)-cyclohexylamin | 4C |

Spektroskopische Daten

Die spektroskopischen Daten einiger ausgewählter Beispiels-Verbindungen sind in den Tabellen 2 bis 5 wiedergegeben.

Tabelle 2

| Beispiels-Nr. | $^1$H NMR ($CDCl_3$) / TMS) $\delta$ [ppm], *J* [Hz] | $^{13}$C NMR ($CDCl_3$) / TMS) $\delta$ [ppm] | IR $\nu$ [$cm^{-1}$] |
|---|---|---|---|
| 44 | 0.74 - 0.83 (m, 1 H, ]-$(CH_2)_4$-[ ), 1.07 - 1.28 (m, 3 H, ]-$(CH_2)_4$-[), 1.57 - 1.70 (m, 3 H, ]-$(CH_2)_4$-[), 1.94 - 2.09 (m, 1 H, CHC*H*CH), 2.12 (6 H, $N(CH_3)_2$), 2.14 - 2.20 (m, 1 H, ]-$(CH_2)_4$-[), 2.29 - 2.36 (m, 1 H, C*H*CHCH), 3.65 (d, 1 H, *J* = 12.8, PhCH), AB-System ($\delta_A$ = 3.65, $\delta_B$ = 3.95, J = 12.8, $CH_2$Ph), 7.11 - 7.40 (m, 10 H. Ar-H). | 24.41, 25.42, 27.49, 31.68 (t, ]-$(CH_2)_4$-[), 41.42 (d, CHCHCHPh), 42.23 (q, N $(CH_3)_2$), 50.75 (t, $CH_2$Ph), 60.44 (d, CHCHCHPh), 73.79 (d, CHPh), 126.50, 126.52, 127.31, 128.01, 128.17, 129.33 (d, CH), 136.36, 141.00 (s, C). | 3444, 1635, 1557, 1452, 1028, 744, 698. |
| 45 | 0.62 - 2.36 (m, 14 H, ]-$(CH_2)_4$-[, C*H*CHCHPh, CHC*H*CH-Ph, ]-$CH_2$-N-$CH_2$[), 3.36 - 3.97 (m, 7 H, $CH_2$Ph, ]-$CH_2$-O-$CH_2$-[, CHPh), 7.11 - 7.37 (m, 10 H, Ar-H). | 25.04, 26.31, 29.49, 33.96 (t, ]-$(CH_2)_4$[), 48.13 (d, CHCHCHPh), 51.54, 52.18 (t, ]-$CH_2$-N-$CH_2$-[, $CH_2$Ph), 61.83 (d, CHCHCHPh), 67.32 (t, -$CH_2$-O$CH_2$-), 67.40 (d, CHPh), 127.25, 128.57, 128.63, 128.71, 128.86 (d, CH), 141.34, 143.13 (s, C). | 3446, 2924, 2852, 1627, 1451, 1383, 1251, 1106, 1070, 700. |

(fortgesetzt)

| Beispiels-Nr. | $^{1}H$ NMR ($CDCl_3$) / TMS) δ [ppm], *J* [Hz] | $^{13}C$ NMR ($CDCl_3$) / TMS) δ [ppm] | IR ν [$cm^{-1}$] |
|---|---|---|---|
| 46 | 0.53 (d, 3 H, *J* = 6.8 Hz, $CHCH_3$), 2.19 (s, 6 H, N $(CH_3)_2$), 2.46 - 2.65 (m, 1 H, $CHCH_3$), 3.23 (d, 1 H, *J* = 9.4, PhCH), AB-System ($\delta_A$ = 3.57, $\delta_B$= 3.71, *J* = 13.1, $CH_2Ph$), 3.93 (d, 1H, *J* = 6.3, PhCH), 7.13 - 7.52 (15 H, Ar-H). | 13.58 (t, $CH_3CH$), 39.37 (d, $CH_3CH$), 42.05 (q, $N(CH_3)_2$), 52.19 (t, $CH_2Ph$), 64.80, 73.07 (d, PhCH), 127.18, 127.97, 128.36, 128.70, 128.77, 128.98. 129.10, 129.93 (d, CH), 136.48, 141.56, 142.63 (s, C). | 3025, 2940, 2791, 1605, 1476, 1444. 1365, 1073, 1028, 754. |

Tabelle 3

| Beispiels-Nr. | $^{1}H$ NMR ($CDCl_3$) /TMS) δ [ppm], J [Hz] | $^{13}C$ NMR ($CDCl_3$) / TMS) δ [ppm] | IR ν ($cm^{-1}$) |
|---|---|---|---|
| 47 | 0.70 - 1.89 (m, 9 H, ]-$(CH_2)_4$-[, CHC*H*CHPh), 2.16 (s, 6 H. N $(CH_3)_2$), 2.43- 2.53 (m, 1 H, CHCHCHPh), 3.40 (d, 1 H, J = 10.9, CHPh), 7.09 -7.42 (m, 5 H, Ar-H). | 24.90. 25.13.30.23.31.83 (t, ]-$(CH_2)_4$-[), 38.17 (q, N $(CH_3)_2$), 45.13 (d, CHCHCHPh), 57.94 (d, CHCHCHPh), 76.65 (d, CHPh), 127.26, 128.03, 129.83 (d, CH), 137.29 (s, C). | 3339, 2955, 2852, 2868.1557.1458. 1452, 1381. |
| 48 | 0.40 - 2.60 (m, 13 H,]-$(CH_2)_4$-[, CHC*H*CHPh, ]-$CH_2$-N-$CH_2$-[ ), 3.16 - 3.96 (m, 5 H, ]-$CH_2$-O-$CH_2$-[, C*H*CHCHPh), 4.19 (d, 1 H, *J* = 10.0, CHPh), 7.21 - 7.56 (m, 5 H, Ar-H). | 25.41. 26.11, 27.26, 37.45 (t, ]-$(CH_2)_4$-[), 44.34 (d, CHCHCH), 51.56 (t, ]-$CH_2$-N-$CH_2$-[), 54.22 (d, CHCHCHPh), 67.40 (t, ]-$CH_2$-O-$CH_2$-[), 67.71 (d, CHPh), 126.83, 127.41, 128.15, 128.59, 129.85 (d, CH), 137.56 (s, C). | 3440, 2921, 2852, 1652, 1456, 1448, 1384, 1113, 1031, 703. |
| 49 | 0.48 (d, 3 H, *J* = 6.8, $CHCH_3$), 2.15 (s, 6 H, $N(CH_3)_2$), 2.65-2.41 (m, 1 H, $CHCH_3$), 3.13 (d, 1 H, *J* = 9.4, N $(CH_3)_2CH$), 4.14 (d. 1 H, J = 6.0, $CHNH_2$), 7.09 - 7.42 (m, 10 H, Ar-H). | 13.00 (q, $CHCH_3$), 40.75 (d, $CHCH_3$), 42.16 (q, $N(CH_3)_2$), 57.84 (d, $N(CH_3)_2CH$), 72.94 (d, $NH_2CH$), 127.09, 127.27, 128.03, 128.13, 128.37, 129.89 (d, CH), 136.54, 145.08 (s, C). | 2950, 2929, 2858, 1729, 1452, 1383, 1185, 1029, |
| 50 | 0.60 - 2.06 (m, 9 H, ]-$(CH_2)_4$-[, CHC*H*CHPh), 2.50 (s, 6 H, N $(CH_3)_2$), 3.10 - 3.19 (m. 2 H, CHPh, C*H*CHCHPh), 7.08 - 7.51 (m, 4 H, Ar-H). | 25.01,25.69,30.01,31.65 (t, ]-$(CH_2)_4$-[), 38.34 (q, N $(CH_3)_2$), 43.47 (d, CHCHCHPh), 69.72 (d, CHPh), 77.98 (d, CHCHCHPh), 127.22, 128.83, 128.95.129.35 (d, CH), 133.27, 135.66 (s, C). | 3430, 2929, 1635, 1438,1062, 750. |
| 51 | 0.60 - 2.06 (m, 9 H, ]-$(CH_2)_4$-[, CHC*H*CHPh), 2.50 (s, 6 H, N $(CH_3)_2$), 3.10 - 3.19 (m, 2 H, CHPh, CHCHCHPh), 7.08 - 7.51 (m, 4 H, Ar-H). | 25.01, 25.69, 30.01, 31.65 (t, ]-$(CH_2)_4$-[), 38.34 (q, N $(CH_3)_2$), 43.47 (d, CHCHCHPh), 69.72 (d, CHPh), 77.98 (d, CHCHCHPh), 127.22, 128.83, 128.95, 129.35 (d, CH), 133.27, 135.66 (s, C). | |

Tabelle 4

| Beispiels-Nr. | $^1$H NMR ($CDCl_3$) / TMS) δ [ppm], J [Hz] | $^{13}$C NMR ($CDCl_3$) / TMS) δ [ppm] | IR ν [$cm^{-1}$] | MS (70 eV) m/z[%] |
|---|---|---|---|---|
| 52 | 0.96 - 2.13 (m, 8 H, ]-$(CH_2)_4$-[, CHC*H*CHPh), 2.17 (s. 6 H, $N(CH_3)_2$), 2.25 - 2.60 (m, 1 H, CHC*H*CHPh), 3.74 - 4.06 (m, 2 H, CHCHCHPh, CHPh), 7.09 - 7.53 (m, 5 H, Ar-H). | 21.86, 24.22,27.45, 32.40 37 (t, ]-$(CH_2)_4$-[), 37.96 (d, CHCHCHPh), 41.25 (q, $N(CH_3)_2$), 68.97 CHCHCHPh), 71.90 (d, CHPh), 127.85, 128.26, 130.24 (d, CH), 136.86 (s, C). | 3405, 2929, 2857, 2782, 1450.1384. 1068, 975, 752, 703. | 232 [$M^+$] (13), 134 (100), 118 (5), 91 (9), 77 (3). |
| 54 | 0.96 -1.88 (m, 8 H, ]-$(CH_2)_4$-[) 2.23 (s, 6 H, $N(CH_3)_2$), 2.31 - 2.56 (m, 1 H, CHC*H*CH), 3.94 - 4.03 (m, 1 H, C*H*CHCHPh), 4.90 (d, 1 H, J = 11.6, CHPh), 7.20 - 7.48 (m, 4 H, Ar-H). | 21.76, 24.63, 27.70, 32.37 (t, ]-$(CH_2)_4$-[), 38.50 (d, CHCHCHPh), 41.49 (q, $N(CH_3)_2$), 62.27 (CHCHCHPh), 72.56 (d, CHPh), 126.42, 128.88, 130.41, 130.56 (d, CH), 132.68, 136.42 (s, C). | 3434, 2929, 2859, 2782, 1643, 1463, 1062, 1035, 975, 754. | 267 [$M^+$] (53), 167 (100), 130 (7). |
| 55 | 0.89 - 1.87 (m, 8 H, ]-$(CH_2)_4$-[) 2.13 (s, 6 H, $N(CH_3)_2$), 2.42 - 2.54 (m, 1 H, CHCHCH), 3.71 - 4.02 (m, 2 H, CHCHCHPh, CHPh), 7.29 - 7.49 (m, 2 H, Ar-H), 8.41 - 8.56 (m, 2 H, Ar-H). | 22.10, 23.72, 27.12, 32.33 (t, ]-$(CH_2)_4$-[), 38.10 (d, CHCHCHPh), 41.16 (q, $N(CH_3)_2$), 66.79 (CHCHCHPh), 71.13 (d, CHPh), 123.43 (d, CH), 128.90 (s, C), 137.13, 149.33, 151.32 (d, CH). | 3417, 2927, 2857, 1646, 1062, 1029, 977. | 235 [$M^+$+ 1], 217 (2), 164 (5),135 (100),119 (4) 92 (2). |
| 57 | 0.95 - 1.94 (m, 8 H, ]-$(CH_2)_4$- D, 2.15 (s, 6 H, $N(CH_3)_2$), 2.48 - 2.56 (m, 1 H, CHCHCH), 3.73 - 4.00 (m, 2 H, C*H*CHCHPh, CHPh), 3.83 (s, 3 H, OMe), 6.94 - 7.01 (m, 2 H, Ar-H). 7.12 (d, 1 H, J = 7.5, Ar-H), 7.28 - 7.33 (m. 1 H, Ar-H). | 21.43,24.92,27.97.32.32 (t, ]-$(CH_2)_4$[), 38.02 (d, CHCHCHPh), 41.42 (q, N $(CH_3)_2$),55.87 (CHCHCHPh), 73.01 (d, CHPh), 111.30, 120.11, 122.38 (s, C), 128.64. 129.65 43 (d, CH), 158.98 (s, C). | 3426,2927, 2857,2784, 1068,975, 752, 703. | 263[$M^+$+ 1](3), 218 (2), 164 (100),148 (12), 121 (7). 91 (8). |

(fortgesetzt)

| Beispiels-Nr. | $^{1}H$ NMR ($CDCl_3$) / TMS) δ [ppm], J [Hz] | $^{13}C$ NMR ($CDCl_3$) / TMS) δ [ppm] | IR ν [$cm^{-1}$] | MS (70 eV) m/z[%] |
|---|---|---|---|---|
| 59 | 0.81 - 1.91 (m, 8 H, )-($CH_2$)$_4$-[, CHC*H*CH), 1.98 (s, 6 H, N($CH_3$)$_2$), 2.20 - 2.46 (m, 2 H, CHCHCH), 3.51 - 3.69 (m, 1 H, C*H*CHCHPh), 4.73 (d, 1 H, J = 11.3, CHPh), 7.29 - 7.41 (m, 2 H, Ar-H), 7.51 - 7.59 (m, 1 H, Ar-H), 7.69 (d, 1 H, J = 8.0). | 22.70, 23.41, 25.92, 32.55 (t, ]-($CH_2$)$_4$-[), 39.03 (d, CHCHCHPh), 40.99 (q, N($CH_3$)$_2$), 60.88 CHCHCHPh), 70.51 (d, CHPh), 124.42 (d, CH), 127.92 (s, C), 128.37, 130.27, 131.56 (d, CH), 152.76 (s, C). | 3417, 2931, 2859, 1527, 1455, 1068, 977. | 277 [$M^+$] (12), 261 (3), 179 (100), 132 (37), 91 (5). |

Tabelle 5

| Beispiels-Nr. | $^{1}H$ NMR ($CDCl_3$) / TMS) δ [ppm], *J* [Hz] | $^{13}C$ NMR ($CDCl_3$) / TMS) δ [ppm] | IR ν [$cm^{-1}$] | MS (70 eV) m/z [%] |
|---|---|---|---|---|
| 53 | 0.53-2.50 (m, 9 H, ]-($CH_2$)$_4$-[, CHCHCH), 2.17 (s, 6 H, N($CH_3$)$_2$), 3.41-3.76 (m, 2 H, C*H*CHCHPh, CHPh), 7.08 - 7.44 (m, 5 H, Ar-H). | 25.03, 26.20, 29.29, 35.37 (t, ]-($CH_2$)$_4$-[), 41.32 (d, CHCHCHPh), 42.75 (q, N($CH_3$)$_2$), 76.60 (CHCHCHPh), 78.00 (d, CHPh), 127.79, 128.17 (d, CH), 137.45 (s, C). | 3421, 2929, 2857, 2782, 1450, 1384, 1062, 1043, 1033, 975. | 232 [$M^+$] (19), 134 (100), 91 (9), 77(3). |
| 56 | 0.57 - 2.07 (m, 9 H, ]-($CH_2$)$_4$-[, CHC*H*CH), 2.14 (s. 6 H, N($CH_3$)$_2$), 3.44 - 3.63 (m. 2 H, C*H*CHCHPh, CHPh), 7.29 - 7.56 (m, 2 H, Ar-H), 8.35 - 8.54 (m, 2 H, Ar-H). | 24.83, 26.03, 29.22, 35.19 (t, ]-($CH_2$)$_4$-[), 41.22 (q, N($CH_3$)$_2$), 42.47 (d, CHCHCHPh), 74.10 (CHCHCHPh), 77.77 (d, CHPh), 123.37(d, CH), 129.63 (s, C), 136.83. 149.32, 151.24 (d, CH). | 3421, 2929, 2857, 1445, 1384, 1070, 1043, 977. | 234 [$M^+$], 164 (5), 135 (100), 91 (5). |
| 58 | 0.61 - 2.52 (m, 9 H, ]-($CH_2$)$_4$-[, CHC*H*CH), 2.17 (s, 6 H, N($CH_3$)$_2$), 3.48 - 3.69 (m, 1 H, C*H*CHCHPh), 3.83 (s, 3 H, $OCH_3$), 4.40 (d, 1 H, J = 11.1, CHPh), 6.92 - 7.30 (m, 4 H, Ar-H). | 25.08, 26.22, 28.87, 35.38 (t, ]-($CH_2$)$_4$-[), 41.59 (d, CHCHCHPh), 42.93 (q, N($CH_3$)$_2$), 55.76 (q, $OCH_3$), 65.42 (CHCHCHPh), 77.98 (d, CHPh), 110.70, 120.40 (d, CH), 122.75 (s, C),127.99, 130.82 (d, CH), 159.16 (s, C). | 3423, 2934, 2857, 2784, 1068, 975, 752, 703. | 262 [$M^+$] (3), 164 (100), 148 (20), 121 (10), 91 (6). |

(fortgesetzt)

| Beispiels-Nr. | $^{1}$H NMR ($CDCl_3$) / TMS) δ [ppm], *J* [Hz] | $^{13}$C NMR ($CDCl_3$) / TMS) δ [ppm] | IR ν [$cm^{-1}$] | MS (70 eV) m/z [%] |
|---|---|---|---|---|
| 60 | 0.92 - 2.49 (m, 9 H, ]-$(CH_2)_4$-[, CHCHCH), 2.07 (s, 6 H, $N(CH_3)_2$), 3.63 - 3.73 (m, 1 H, CHCHCHPh), 4.42 (d, 1 H, J = 10.6 Hz, CHPh), 7.33 - 7.81 (m, 4 H, Ar-H). | 24.75, 26.03, 28.42, 35.11 (t, ]-$(CH_2)_4$-[), 41.40 (q, $N(CH_3)2$), 43.02 (d, CHCHCHPh), 67.78 (CHCHCHPh), 77.54 (d, CHPh), 124.41, 128.54, 129.37 (s, C), 130.54, 131.81 (d, CH), 152.45 (s, C). | 3415, 2936, 2864, 1523, 1455, 1068, 977. | 277 [$M^+$] (20), 179 (100), 132 (37), 91 (30). |

**Pharmakologische Untersuchungen**

Analgesieprüfung im Writhing-Test an der Maus

Die Untersuchung auf analgetische Wirksamkeit wurde im Phenylchinoninduzierten Writhing an der Maus (modifiziert nach I.C. Hendershot und J. Forsaith (1959) J. Pharmacol. Exp. Ther. 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhielten. Alle Substanzen wurden in der Standarddosierung von 10 mg/kg getestet. Die prozentuale Hemmung (%Hemmung) der Writhingreaktion durch eine Substanz wurde nach folgender Formel berechnet:

$$\% \text{ Hemmung} = 100 - \frac{\text{Writhingreaktionen der behandelten Tiere}}{\text{Writhingreaktionen der Kontrolltiere}} * 100$$

Alle untersuchten erfindungsgemäßen Verbindungen zeigten eine ausgeprägte analgetische Wirkung. Die Ergebnisse sind in der nachfolgenden Tabelle 6 zusammengefaßt.

Tabelle 6

| Beispiels-Nr. | %Hemmung der Writhingreaktion (Dosierung in mg/kg intravenös) | |
|---|---|---|
| 1 | 54 | (10) |
| 2 | 67 | (10) |
| 3 | 85 | (10) |
| 4 | 34 | (10) |
| 5 | 49 | (10) |
| 6 | 62 | (10) |
| 7 | 56 | (10) |
| 8 | 40 | (10) |

(fortgesetzt)

| Beispiels-Nr. | %Hemmung der Writhingreaktion (Dosierung in mg/kg intravenös) | |
|---|---|---|
| 9 | 75 | (10) |
| 10 | 59 | (10) |

Pharmazeutische Formulierung eines erfindungsgemäßen Arzneimittels

1 g des Hydrochlorids von (syn,syn)-2-Chlor-N-[2-(Dimethylaminopyridin-3-ylmethyl)cyclohexyl]-benzamid wurde in 1 l Wasser für Injektionszwecke bei Raumtemperatur gelöst und anschließend durch Zugabe von Natriumchlorid auf isotone Bedingungen eingestellt.

## Patentansprüche

1. Verbindung der allgemeinen Struktur (I), oder eines ihrer pharmazeutisch annehmbaren Salze,

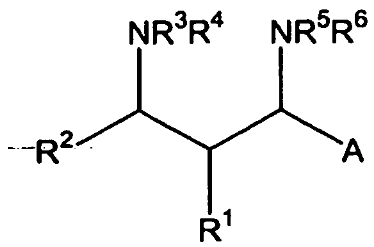

I ,

worin

$R^1$ Methyl oder Ethyl bedeutet,
$R^2$ Methyl, Ethyl oder Phenyl bedeutet, oder
$R^1$ und $R^2$ zusammen $-(CH_2)_4-$ bilden;
$R^3$ H, n-Propyl, $-CH_2$-Phenyl oder C(=O)-$R^7$ bedeutet;
$R^4$ H bedeutet;
$R^5$ und $R^6$ jeweils Methyl bedeuten oder zusammen $-(CH_2)_2-O-(CH_2)_2-$ bilden;
A Phenyl, 2-Chlorphenyl, 2-Methoxyphenyl, 2-Nitrophenyl oder Pyridin-3-yl bedeutet; und
$R^7$ Methyl, Phenyl, 2-Fluorphenyl, 2-Chlorphenyl oder 2-Methylphenyl bedeutet,

wobei

- Benzyl-[2-(dimethylamino-phenyl-methyl)-cyclohexyl]-amin
- (2-Methyl-1,3-diphenyl-3-piperidin-1-yl-propyl)-propyl-amin ausgenommen sind.

2. Verbindung nach Anspruch 1 oder eines ihrer pharmazeutisch annehmbaren Salze, wobei die Verbindung der allgemeinen Struktur (I) als Diastereomer der Formel (syn,anti-I)

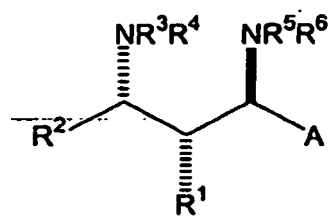

syn,anti-I

und gegebenenfalls enantiomerenrein vorliegt.

3. Verbindung nach Anspruch 1 oder eines ihrer pharmazeutisch annehmbaren Salze, wobei die Verbindung der allgemeinen Struktur-(1) als Diastereomer der Formel (anti,anti-I)

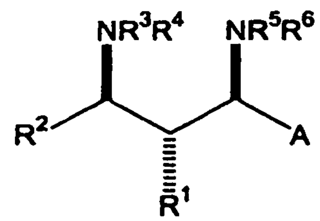

anti,anti-I

und gegebenenfalls enantiomerenrein vorliegt.

4. Verbindung nach Anspruch 1 oder eines ihrer pharmazeutisch annehmbaren Salze, wobei die Verbindung der allgemeinen Struktur (I) als Diastereomer der Formel (anti,syn-I)

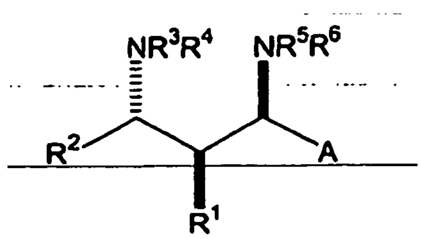

anti,syn-I

und gegebenenfalls enantiomerenrein vorliegt.

5. Verbindung nach Anspruch 1 oder eines ihrer pharmazeutisch annehmbaren Salze, wobei die Verbindung der allgemeinen Struktur (I) als Diastereomer der Formel (syn,syn-I)

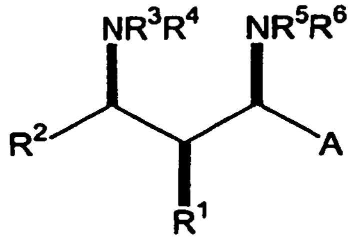

syn,syn-I

und gegebenenfalls enantiomerenrein vorliegt.

**6.** Verbindung nach Anspruch 1, oder eines ihrer pharmazeutisch annehmbaren Salze, die ausgewählt ist aus:

- (syn,syn)-N-[2-(Dimethylaminopyridin-3-ylmethyl)cyctohexyl]-benzamid oder sein Hydrochlorid
- (syn,syn)-2-(Dimethylaminopyridin-3-ylmethyl)cyclohexylamin oder sein Hydrochlorid
- (syn,syn)-N-[2-(Dimethylaminopyridin-3-ylmethyl)cyclohexyl]-2-fluorbenzamid oder sein Hydrochlorid
- (syn,syn)-2-Chlor-N-[2-(Dimethylaminopyridin-3-ylmethyl)cyclohexyl]-benzamid oder sein Hydrochlorid
- (anti,anti)-N-[2-(Dimethylaminopyridin-3-ylmethyl)cyclohexyl]-benzamid oder sein Hydrochlorid
- (anti,anti)-2-(Dimethylaminopyridin-3-ylmethyl)cyclohexylamin oder sein Hydrochlorid
- (anti,anti)-N-[2-(Dimethylaminopyridin-3-ylmethyl)cyclohexyl]-2-fluorbenzamid oder sein Hydrochlorid
- (anti,anti)-2-Chlor-N-[2-(dimethylaminopyridin-3-ylmethyl)cyclohexyl]benzamid oder sein Hydrochlorid
- (anti,anti)-N-[2-(Dimethylaminopyridin-3-ylmethyl)cyclohexyl]-2-methylbenzamid oder sein Hydrochlorid
- (syn,syn)-N-[2-(Dimethylaminopyridin-3-ylmethyl)cyclohexyl]-2-methylbenzamid oder sein Hydrochlorid
- (syn,syn)-N-[2-(Dimethylaminopyridin-3-ylmethyl)cyclohexyl]acetamid oder sein Hydrochlorid
- (anti,anti)-N-[2-(Dimethylaminopyridin-3-ylmethyl)cyclohexyl]acetamid oder sein Hydrochlorid
- (syn,syn)-N-[2-(Dimethylaminophenylmethyl)cyclohexyl]-2-fluorbenzamid oder sein Hydrochlorid
- (syn,syn)-2-(Dimethylaminophenylmethyl)cyclohexylamin oder sein Hydrochlorid
- (syn,syn)-N-[2-(Dimethylamino-phenyl-methyl)-cyclohexyl]-acetamid oder sein Hydrochlorid
- (syn,syn)-N-[2-(Dimethylamino-phenyl-methyl)-cyclohexyl]-benzamid oder sein Hydrochlorid
- (syn,syn)-2-Chlor-N-[2-(dimethylamino-phenyl-methyl)-cyclohexyl]-benzamid oder sein Hydrochlorid
- (syn,syn)-N-[2-(Dimethylamino-phenyl-methyl)-cyclohexyl]-2-methyl-benzamid oder-sein-Hydrochlorid
- (anti,anti)-N-[2-(Dimethytamino-phenyl-methyl)-cyclohexyl]-acetamid oder sein Hydrochlorid
- (anti,anti)-2-(Dimethylamino-phenyl-methyl)-cyclohexylamin oder sein Hydrochlorid
- (anti,anti)-N-[2-(Dimethylamino-phenyl-methyl)-cyclohexyl]-benzamid oder sein Hydrochlorid
- (anti,anti)-N-(2-Dimethylamino-phenyl-methyl)-cyclohexyl]-2-methyl-benzamid oder sein Hydrochlorid
- (syn,syn)-2-Chlor N-{2-[(2-chlor-phenyl)-dimethylamino-methyl]-cyclohexyl}-benzamid oder sein Hydrochlorid
- (syn,syn)-2-[(2-Chlor-phenyl)-dimethylamino-methyl]-cyclohexylamin oder sein Hydrochlorid
- (anti,anti)-2-Chlor-N-{2-[(2-chlor-phenyl)-dimethylamino-methyl]-cyclohexyl}-benzamid oder sein Hydrochlorid
- (anti,anti)-2-[(2-Chlor-phenyl)-dimethylamino-methyl]-cyclohexylamin oder sein Hydrochlorid
- (syn,syn)-N-{2-[(2-Chlor-phenyl)-dimethylamino-methyl]-cyclohexyl}-2-fluor-benzamid oder sein Hydrochlorid
- (anti,anti)-N-{2-[Dimethylamino-(2-nitro-phenyl)-methyl]-cyclohexyl}-benzamide oder sein Hydrochlorid
- (anti,anti)-2-[Dimethylamino-(2-nitro-phenyl)-methyl]-cyclohexylamin oder sein Hydrochlorid
- (anti,anti)-N-{2-[Dimethylamino-(2-nitro-phenyl)methyl]-cyclohexyl}-2-fluor-benzamid oder sein Hydrochlorid
- (anti,anti)-2-Chlor-N-{2-[dimethylamino-(2-nitro-phenyl)-methyl]-cyclohexyl}-benzamid oder sein Hydrochlorid
- (anti,anti)-N-{2-[Dimethylamino-(2-nitro-phenyl)-methyl]-cyclohexyl}-2-methyl-benzamid oder sein Hydrochlorid
- (syn,syn)-N-{2-[Dimethylamino-(2-nitro-phenyl)-methyl]-cyclohexyl}-acetamid oder sein Hydrochlorid
- (syn,syn)-N-2-[Dimethylamino-(2-nitro-phenyl)-methyl]-cyclohexylamin oder sein Hydrochlorid
- (anti,anti)-N-{2-[(2-Chlor-phenyl)-dimethylamino-methyl]-cyclohexyl}-acetamid oder sein Hydrochlorid
- (syn,anti)-2-(Dimethylamino-phenyl-methyl)cyclohexylamin
- (syn,anti)-N-[2-(Dimethylamino-phenyl-methyl)-cyclohexyl]-benzamid
- (anti,anti)-N-{2-[Dimethylamino-(2-methoxy-phenyl)-methyl]-cyclohexyl}-benzamid
- (anti,anti)-N-{2-[Dimethylamino-(2-nitro-phenyl)-methyl]-cyclohexyl}-benzamid

- (anti,anti)-N-{2-[(2-Chlor phenyl)-dimethylamino-methyl]-cyclohexyl}-benzamid
- (anti,anti)-N-{2-[Dimethylamino-(2-methoxy-phenyl)-methyl]-cyclohexyl}-acetamid
- (anti,anti)-2-[Dimethylamino-(2-methoxy-phenyl)-methyl]-cyclohexylamin
- (anti,anti)-N-{2-[(2-Chlor-phenyl)-dimethylamino-methyl]-cyclohexyl}-acetamid
- (anti,anti)-2-[(2-Chlor-phenyl)-dimethylamino-methyl]-cyclohexylamin
- (anti,anti)-N-{2-[Dimethylamino-(2-nitro-phenyl)-methyl]-cyclohexyl}-acetamid
- (anti,anti)-2-[Dimethylamino-(2-nitro-phenyl)-methyl]-cyclohexylamin
- (syn,syn)-2-(Dimethylamino-phenyl-methyl)-cyclohexylamin
- (syn,syn)-2-[(2-Chlor-phenyl)-dimethylamino-methyl]-cyclohexylamin
- (anti,anti)-2-Chlor-N-(3-dimethylamino-1-ethyl-2-methyl-3-phenylpropyl)-benzamid
- (anti,anti)-3-Dimethylamino-1-ethyl-2-methyl-3-phenyl-propylamin
- (syn,anti)-2-(Dimethylamino-phenyl-methyl)-cyclohexyl-N-(n-propyl)-amin
- (syn,anti)-2-(Morpholin-4-yl-phenyl-methyl)-cyclohexyl-N-(n-propyl)-amin
- (syn,anti)-2,N,N-Trimethyl-1,3-diphenyl-N'-propyl-propan-1,3-diamin
- (syn,anti)-2-(Dimethylamino-phenyl-methyl)-cyclohexyl-N-benzyl-amin
- (syn,anti)-2-(Morpholin-4-yl-phenyl-methyl)-cyclohexyl-N-benzyl-amin
- (syn,anti)-2,N,N-Trimethyl-1,3-diphenyl-N'-benzyl-propan-1,3-diamin
- (syn,anti)-2-(Dimethylamino-phenyl-methyl)-cyclohexylamin
- (syn,anti)-2-(Morpholin-4-yl-phenyl-methyl)-cyclohexylamin
- (syn,anti)-2,N,N-Trimethyl-1,3-diphenyl-propan-1,3-diamin
- (syn,anti)-2-[(2-Chlorphenyl)-dimethylamino-methyl]-cyclohexylamin
- (anti,anti)-2-[(2-Chlorphenyl)-dimethylamino-methyl]-cyclohexylamin
- (syn,syn)-2-(Dimethylamino-phenyl-methyl)-cyclohexylamin
- (anti,anti)-2-(Dimethylamino-phenyl-methyl)-cyclohexylamin
- (syn,syn)-2-[(2-Chlorphenyl)-dimethylamino-methyl]-cyclohexylamin
- (syn,syn)-2-(Dimethylamino-pyridin-3-yl-methyl)-cyclohexylamin
- (anti,anti)-2-(Dimethylamino-pyridin-3-yl-methyl)-cyclohexylamin
- (syn,syn)-2-(Dimethylamino-(2-methoxyphenyl)-methyl)-cyclohexylamin
- (anti,anti)-2-(Dimethylamino-(2-methoxyphenyl)-methyl)-cyclohexylamin
- (syn,syn)-2-(Dimethylamino-(2-nitrophenyl)-methyl)-cyclohexylamin
- (anti,anti)-2-(Dimethylamino-(2-nitrophenyl)-methyl)-cyclohexylamin

**7.** Verfahren zur Herstellung einer Verbindung nach Anspruch 1 der allgemeinen Struktur (I), oder eines ihrer pharmazeutisch annehmbaren Salze,

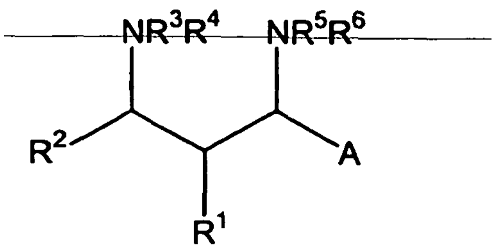

I

worin

$R^1$ Methyl oder Ethyl bedeutet,
$R^2$ Methyl, Ethyl oder Phenyl bedeutet, oder
$R^1$ und $R^2$ zusammen $-(CH_2)_4-$ bilden;
$R^3$ H, n-Propyl oder $-CH_2$-Phenyl bedeutet;
$R^4$ H bedeutet;
$R^5$ und $R^6$ jeweils Methyl bedeuten oder zusammen $-(CH_2)_2-O-(CH_2)_2-$ bilden; und
A Phenyl, 2-Chlorphenyl, 2-Methoxyphenyl, 2-Nitrophenyl oder Pyridin-3-yl bedeutet,

**dadurch gekennzeichnet, daß** ein Imin der allgemeinen Struktur (II)

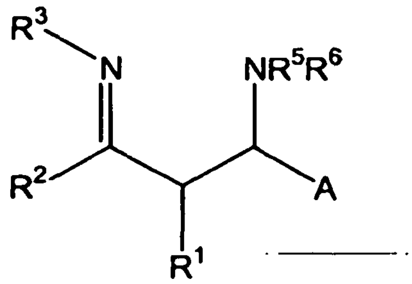

II ,

worin $R^1$, $R^2$, $R^3$, $R^5$, $R^6$ und A wie oben in diesem Anspruch definiert sind,
mit einem geeigneten Reduktionsmittel umgesetzt wird.

8. Verfahren nach Anspruch 7, **dadurch gekennzeichnet, daß** das Reduktionsmittel ein komplexes Hydrid ist.

9. Verfahren nach einem der Ansprüche 7 oder 8 zur diastereoselektiven Herstellung einer Verbindung der allgemeinen Struktur (anti,anti-I), oder eines ihrer pharmazeutisch annehmbaren Salze,

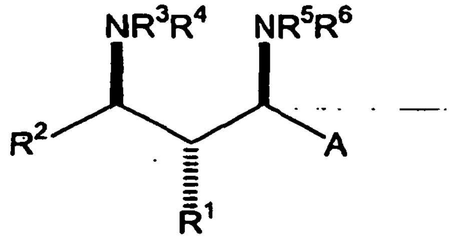

anti,anti-I ,

worin $R^1$, $R^2$, $R^3$, $R^4$, $R^5$, $R^6$ und A wie in Anspruch 7 definiert sind, **dadurch gekennzeichnet, daß** ein Imin der allgemeinen Struktur (anti-II)

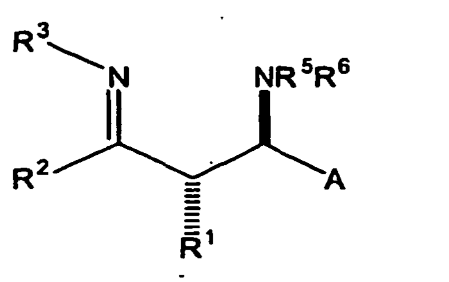

anti-II ,

worin $R^1$, $R^2$, $R^3$, $R^5$, $R^6$ und A wie oben in diesem Anspruch definiert sind,
mit einem geeigneten Reduktionsmittel, insbesondere Zinkcyanoborhydrid ($ZnCNBH_3$), $LiBH_4$, $NaBH_4$, $NaBH_3CN$ oder $NaBH(OC(=O)CH_3)_3$, in einem alkoholischen Lösungsmittel umgesetzt wird.

10. Verfahren nach Anspruch 9, **dadurch gekennzeichnet, daß** die Umsetzung mit Zinkcyanoborhydrid, $LiBH_4$, $NaBH_4$, $NABH_3CN$ oder $NaBH(OC(=O)CH_3)_3$ in Methanol unter Erwärmen von 0 ˚C bis Raumtemperatur über 8 bis 24 h,

vorzugsweise über 10 bis 14 h, durchgeführTwird.

**11.** Verfahren nach einem der Ansprüche 7 oder 8 zur diastereoselektiven Herstellung einer Verbindung der allgemeinen Struktur (syn,syn-1), oder eines ihrer pharmazeutisch annehmbaren Salze,

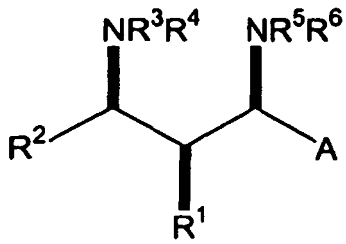

**syn,syn-I** ,

worin $R^1$, $R^2$, $R^3$, $R^4$, $R^5$, $R^6$ und A wie in Anspruch 7 definiert sind,
**dadurch gekennzeichnet, daß**
ein Imin der allgemeinen Struktur (anti-II)

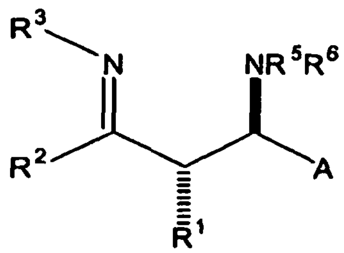

**anti-II** ,

worin $R^1$, $R^2$, $R^3$, $R^5$, $R^6$ und A wie oben in diesem Anspruch definiert sind,
mit einem geeigneten Reduktionsmittel, insbesondere L-Selectride oder Diisobutylaluminiumhydrid, in einem etherischen Lösungsmittel umgesetzt wird.

**12.** Verfahren nach Anspruch 11, **dadurch gekennzeichnet, daß** die Umsetzung mit L-Selectride oder Diisobutylaluminiumhydrid in 8 bis 24 h, vorzugsweise über 10 bis 14 h, durchgeführt wird.

**13.** Verfahren nach einem der Ansprüche 7 bis 12, **dadurch gekennzeichnet, daß** das Imin der allgemeinen Struktur (II) bzw. (anti-II) durch Umsetzung der Mannich-Base (III) bzw. (anti-III)

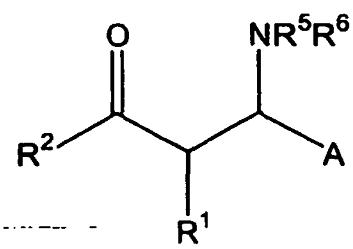

**III**

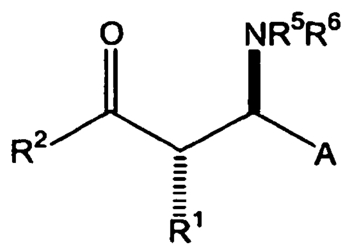

**anti-III** ,

worin $R^1$, $R^2$, $R^5$, $R^6$ und A wie in Anspruch 7 definiert sind,

mit einem Amin der Formel $R^3NH_2$, wenn $R^3$ in Formel (II) bzw. (anti-II) nicht H bedeutet, und mit Ammoniumacetat, wenn $R^3$ in Formel (II) bzw. (anti-II) H bedeutet, in einem etherischen oder alkoholischen Lösungsmittel hergestellt wird.

**14.** Verfahren zur Herstellung einer Verbindung der allgemeinen Struktur (I), oder eines ihrer pharmazeutisch annehmbaren Salze,

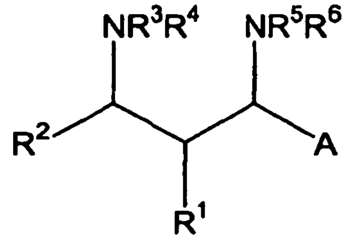

I ,

worin

$R^1$ Methyl oder Ethyl bedeutet,
$R^2$ Methyl, Ethyl oder Phenyl bedeutet, oder
$R^1$ und $R^2$ zusammen $-(CH_2)_4-$ bilden;
$R^3$ und $R^4$ H bedeuten;
$R^5$ und $R^6$ jeweils Methyl bedeuten oder zusammen $-(CH_2)_2-O-(CH_2)_2-$ bilden; und
A Phenyl, 2-Chlorphenyl, 2-Methoxyphenyl, 2-Nitrophenyl oder Pyridin-3-yl bedeutet,

**gekennzeichnet durch** die Verfahrensschritte:

(a) Überführung eines Aminoalkohols der allgemeinen Struktur (IV)

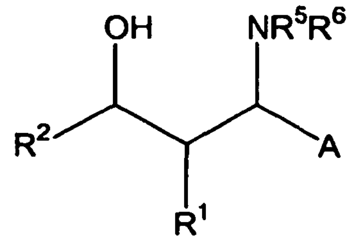

IV ,

worin $R^1$, $R^2$, $R^5$, $R^6$ und A wie oben in diesem Anspruch definiert sind,
in die Verbindung der allgemeinen Struktur (V)

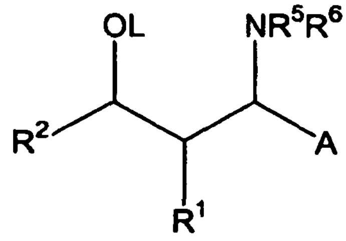

V ,

worin L Mesyl oder Tosyl bedeutet;

(b) Überführung der Verbindung der allgemeinen Struktur (V) in ein Azid der allgemeinen Struktur (VI)

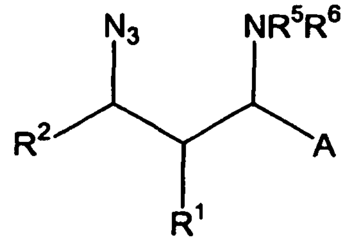

VI ;

und

(c) Reduktion des Azids der allgemeinen Struktur (VI) zu dem Diamin der allgemeinen Struktur (I).

**15.** Verfahren nach Anspruch 14 zur diastereoselektiven Herstellung einer Verbindung der allgemeinen Struktur (syn, anti-I) bzw. (anti,anti-I), oder eines ihrer pharmazeutisch annehmbaren Salze,

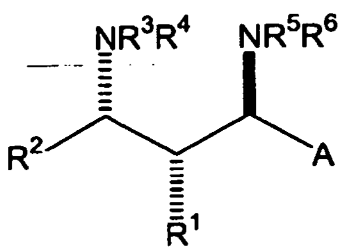

syn,anti-I

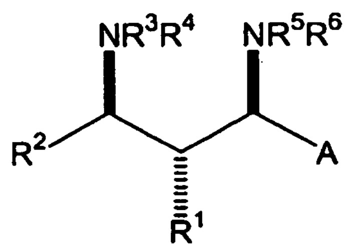

anti,anti-I ,

worin $R^1$, $R^2$, $R^3$, $R^4$, $R^5$, $R^6$ und A wie in Anspruch 14 definiert sind, **gekennzeichnet durch** die Verfahrensschritte:

(a') Überführung eines Aminoalkohols der allgemeinen Struktur (anti,anti-IV) bzw. (syn,anti-IV)

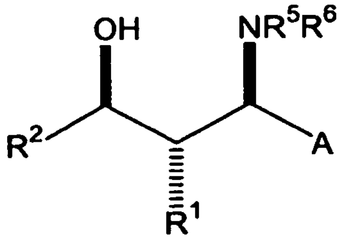

anti,anti-IV

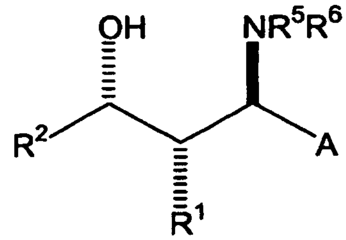

syn,anti-IV ,

worin $R^1$, $R^2$, $R^5$, $R^6$ und A wie oben in diesem Anspruch definiert sind, in die Verbindung der allgemeinen Struktur (anti,anti-V) bzw. (syn,anti-V)

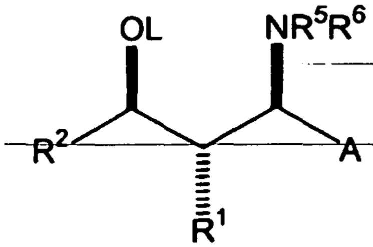

anti,anti-V

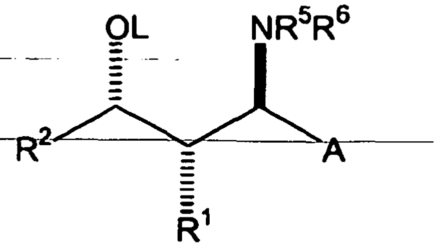

syn,anti-V

worin L Mesyl oder Tosyl bedeutet;
(b') Überführung der Verbindung der allgemeinen Struktur (anti,anti-V) bzw. (syn,anti-V) in ein Azid der allgemeinen Struktur (syn,anti-VI) bzw. (anti,anti-VI)

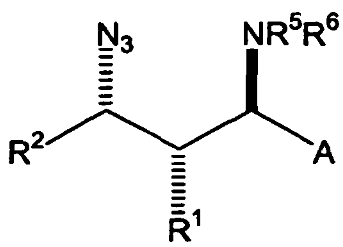

syn,anti-VI

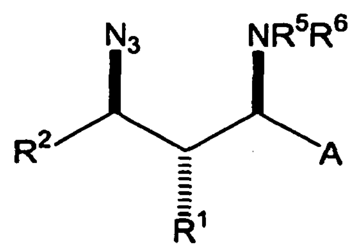

anti,anti-VI ;

und
(c') Reduktion des Azids der allgemeinen Struktur (syn,anti-VI) bzw. (anti,anti-VI) zu dem Diamin der allgemeinen Struktur (syn,anti-I) bzw. (anti,anti-I).

**16.** Verfahren zur Herstellung einer Verbindung der allgemeinen Struktur (syn,anti-I), oder eines ihrer pharmazeutisch annehmbaren Salze,

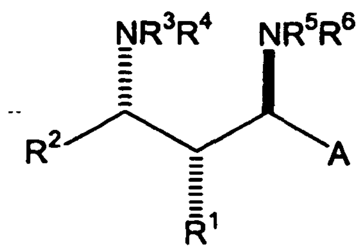

**syn,anti-I** ,

nach Anspruch 2, worin

$R^1$ Methyl oder Ethyl bedeutet,
$R^2$ Methyl, Ethyl oder Phenyl bedeutet, oder
$R^1$ und $R^2$ zusammen $-(CH_2)_4-$ bilden;
$R^3$ H, n-Propyl oder $-CH_2$-Phenyl bedeutet;
$R^4$ H bedeutet;
$R^5$ und $R^6$ jeweils Methyl bedeuten oder zusammen $-(CH_2)_2-O-(CH_2)_2-$ bilden; und
A Phenyl, 2-Chlorphenyl, 2-Methoxyphenyl, 2-Nitrophenyl oder Pyridin-3-yl bedeutet,

**gekennzeichnet durch** die Verfahrensschritte

(aa) Umsetzung eines Imins der allgemeinen Struktur (VII)

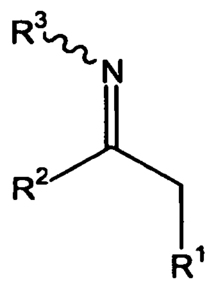

**VII** ,

worin $R^1$, $R^2$ und $R^3$ wie oben in diesem Anspruch definiert sind, mit einem Iminiumsalz der allgemeinen Struktur (VIII)

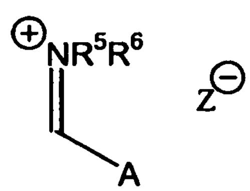

**VIII** ;

worin. $R^5$, $R^6$ und A wie oben in diesem Anspruch definiert sind und $Z^{\ominus}$ ein geeignetes Gegenion darstellt; und
(bb) anschließende Reduktion.

**17.** Verfahren nach Anspruch 16 zur Herstellung einer Verbindung der allgemeinen Struktur (syn,anti-I)

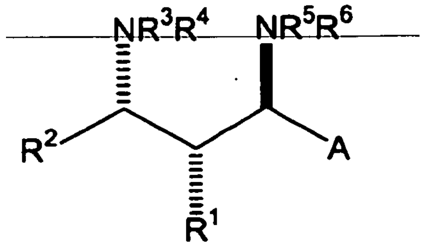

**syn,anti-I** ,

worin $R^1$, $R^2$, $R^4$, $R^5$, $R^6$ und A wie in Anspruch 16 definiert sind und $R^3$ H bedeutet, **dadurch gekennzeichnet, daß** in einem weiteren Verfahrensschritt (cc) eine Verbindung der allgemeinen Struktur (syn,anti-1), worin $R^1$, $R^2$, $R^4$, $R^5$, $R^6$ und A wie in Anspruch 16 definiert sind und $R^3$ -($CH_2$)-Phenyl bedeutet, wobei Phenyl mit $C_{1-6}$-Alkyl substituiert sein kann, in Gegenwart eines Übergangmetalls, das aus Platin, Palladium und Nickel ausgewählt ist, mit Wasserstoff ($H_2$) umgesetzt wird.

**18.** Verfahren zur Herstellung einer Verbindung der allgemeinen Struktur (I), oder eines ihrer pharmazeutisch annehmbaren Salze,

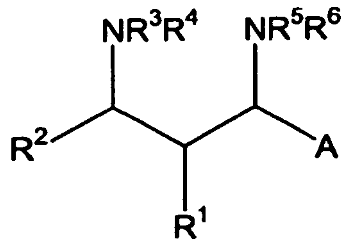

**I** ,

worin

$R^1$ Methyl oder Ethyl bedeutet,
$R^2$ Methyl, Ethyl oder Phenyl bedeutet, oder
$R^1$ und $R^2$ zusammen -$(CH_2)_4$- bilden;
$R^3$ C(=O)-$R^7$ bedeutet;
$R^4$ H bedeutet;
$R^5$ und $R^6$ jeweils Methyl bedeuten oder zusammen -$(CH_2)_2$-O-$(CH_2)_2$- bilden;
A Phenyl, 2-Chlorphenyl, 2-Methoxyphenyl, 2-Nitrophenyl oder Pyridin-3-yl bedeutet; und
$R^7$ Methyl, Phenyl, 2-Fluorphenyl, 2-Chlorphenyl oder 2-Methylphenyl bedeutet,

und die als Racemat, in Form eines oder mehrerer Diastereomeren oder eines oder mehrerer Enantiomeren vorliegt, **dadurch gekennzeichnet, daß** eine Verbindung der allgemeinen Struktur (I), worin $R^1$, $R^2$, $R^4$, $R^5$, $R^6$ und A wie oben in diesem Anspruch definiert sind und $R^3$ H bedeutet, und die als Racemat, in Form eines oder mehrerer Diastereomeren oder eines oder mehrerer Enantiomeren vorliegt, mit einem Acylierungsreagenz umgesetzt wird.

**19.** Verfahren nach Anspruch 18, **dadurch gekennzeichnet, daß** das Acylierungsreagenz ein Säurechlorid der allgemeinen Formel $R^7$-C(=O)-Cl ist, worin $R^7$ Methyl, Phenyl, 2-Fluorphenyl, 2-Chlorphenyl oder 2-Methylphenyl bedeutet.

**20.** Arzneimittel, enthaltend eine Verbindung der allgemeinen Struktur (1) oder eines ihrer pharmazeutisch annehmbaren

Salze, die/das als Racemat, in Form eines oder mehrerer Diastereomeren oder eines oder mehrerer Enantiomeren vorliegt,

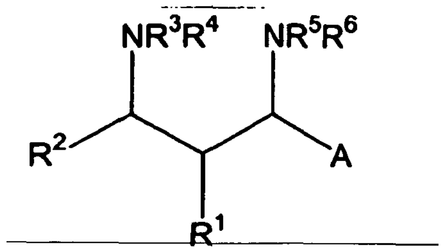

I ,

worin

$R^1$ Methyl oder Ethyl bedeutet,
$R^2$ Methyl, Ethyl oder Phenyl bedeutet, oder
$R^1$ und $R^2$ zusammen $-(CH_2)_4-$ bilden;
$R^3$ H, n-Propyl, $-CH_2$-Phenyl oder C(=O)-$R^7$ bedeutet;
$R^4$ H bedeutet;
$R^5$ und $R^6$ jeweils Methyl bedeuten oder zusammen $-(CH_2)_2-O-(CH_2)_2-$ bilden;
A Phenyl, 2-Chlorphenyl, 2-Methoxyphenyl, 2-Nitrophenyl oder Pyridin-3-yl bedeutet; und
$R^7$ Methyl, Phenyl, 2-Fluorphenyl, 2-Chlorphenyl oder 2-Methylphenyl bedeutet.

**21.** Verwendung einer Verbindung der allgemeinen Struktur (I) oder eines ihrer pharmazeutisch annehmbaren Salze, die/das als Racemat, in Form eines oder mehrerer Diastereomeren oder eines oder mehrerer Enantiomeren vorliegt,

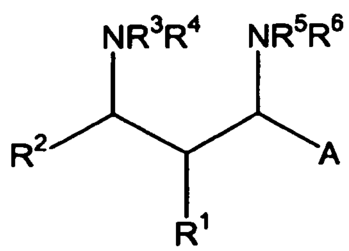

I ,

worin

$R^1$ Methyl oder Ethyl bedeutet,
$R^2$ Methyl, Ethyl oder Phenyl bedeutet, oder
$R^1$ und $R^2$ zusammen $-(CH_2)_4-$ bilden;
$R^3$ H, n-Propyl, $-CH_2$-Phenyl oder C(=O)-$R^7$ bedeutet;
$R^4$ H bedeutet;
$R^5$ und $R^6$ jeweils Methyl bedeuten oder zusammen $-(CH_2)_2-O-(CH_2)_2-$bilden;
A Phenyl, 2-Chlorphenyl, 2-Methoxyphenyl, 2-Nitrophenyl oder Pyridin-3-yl bedeutet; und
$R^7$ Methyl, Phenyl, 2-Fluorphenyl, 2-Chlorphenyl oder 2-Methylphenyl bedeutet,

zur Herstellung eines Medikaments zur Therapie und/oder Prophylaxe von Schmerz.

**22.** Verwendung einer Verbindung der allgemeinen Struktur (I) oder eines ihrer pharmazeutisch annehmbaren Salze,

die/das als als Racemat, in Form eines oder mehrerer Diastereomeren oder eines oder mehrerer Enantiomeren vorliegt,

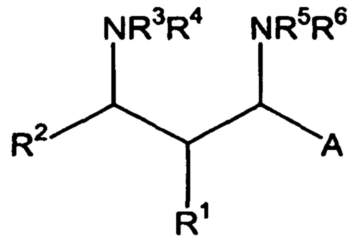

I ,

worin

$R^1$ Methyl oder Ethyl bedeutet,
$R^2$ Methyl, Ethyl oder Phenyl bedeutet, oder
$R^1$ und $R^2$ zusammen $-(CH_2)_4-$ bilden;
$R^3$ H, n-Propyl, $-CH_2$-Phenyl oder C(=O)-$R^7$ bedeutet;
$R^4$ H bedeutet;
$R^5$ und $R^6$ jeweils Methyl bedeuten oder zusammen $-(CH_2)_2-O-(CH_2)_2-$ bilden;
A Phenyl, 2-Chlorphenyl, 2-Methoxyphenyl, 2-Nitrophenyl oder Pyridin-3-yl bedeutet; und
$R^7$ Methyl, Phenyl, 2-Fluorphenyl, 2-Chlorphenyl oder 2-Methylphenyl bedeutet,

zur Herstellung eines Medikaments zur Therapie und/oder Prophylaxe von Harninkontinenz, Juckreiz, Tinnitus aurium und/oder Diarrhoe.

## Claims

1. Compound of the general structure (I), or one of its pharmaceutically acceptable salts,

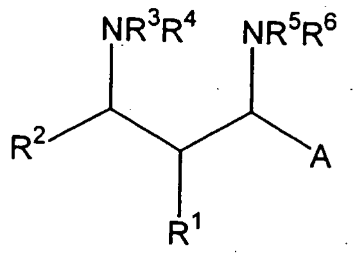

I ,

wherein

$R^1$ denotes methyl or ethyl,
$R^2$ denotes methyl, ethyl or phenyl, or
$R^1$ and $R^2$ together form $-(CH_2)_4-$ ;
$R^3$ denotes H, n-propyl, $-CH_2$-phenyl or C(=O)-$R^7$;
$R^4$ denotes H;
$R^5$ and $R^6$ each denote methyl or together form $-(CH_2)_2-O-(CH_2)_2-$;
A denotes phenyl, 2-chlorophenyl, 2-methoxyphenyl, 2-nitrophenyl or pyridin-3-yl; and

$R^7$ denotes methyl, phenyl, 2-fluorophenyl, 2-chlorophenyl or 2-methylphenyl,

excluding

- benzyl-[2-(dimethylamino-phenyl-methyl)-cyclohexyl]-amine and
- (2-methyl-1,3-diphenyl-3-piperidin-1-yl-propyl)-propyl-amine.

**2.** Compound according to claim 1 or one of its pharmaceutically acceptable salts, wherein the compound of the general structure (I) is present as the diastereomer of the formula (syn,anti-I)

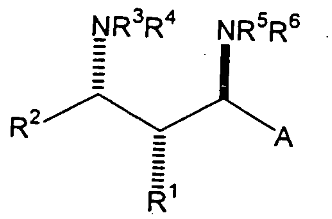

**syn,anti-I**

and optionally in the enantiomerically pure form.

**3.** Compound according to claim 1 or one of its pharmaceutically acceptable salts, wherein the compound of the general structure (I) is present as the diastereomer of the formula (anti,anti-I)

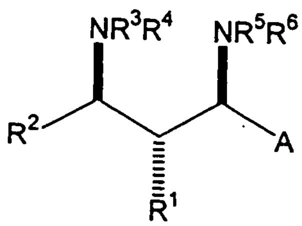

**anti,anti-I**

and optionally in the enantiomerically pure form.

**4.** Compound according to claim 1 or one of its pharmaceutically acceptable salts, wherein the compound of the general structure (I) is present as the diastereomer of the formula (anti,syn-I)

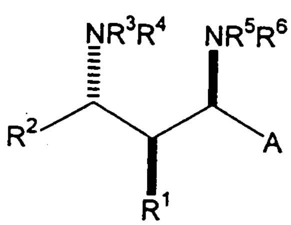

**anti,syn-I**

and optionally in the enantiomerically pure form.

**5.** Compound according to claim 1 or one of its pharmaceutically acceptable salts, wherein the compound of the general structure (I) is present as the diastereomer of the formula (syn,syn-I)

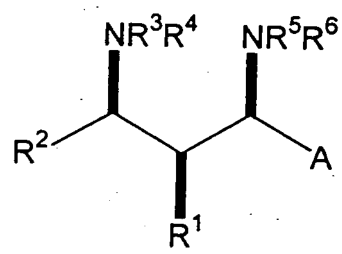

**syn,syn-I**

and optionally in the enantiomerically pure form.

**6.** Compound according to claim 1, or one of its pharmaceutically acceptable salts, which is chosen from:

1 • (syn,syn)-N-[2-(dimethylaminopyridin-3-ylmethyl)cyclohexyl]-benzamide or its hydrochloride
2 • (syn,syn)-2-(dimethylaminopyridin-3-ylmethyl)cyclohexylamine or its hydrochloride
3 • (syn,syn)-N-[2-(dimethylaminopyridin-3-ylmethyl)cyclohexyl]-2-fluorobenzamide or its hydrochloride
4 • (syn,syn)-2-chloro-N-[2-(dimethylaminopyridin-3-ylmethyl)cyclohexyl]-benzamide or its hydrochloride
5 • (anti,anti)-N-[2-(dimethylaminopyridin-3-ylmethyl)cyclohexyl]-benzamide or its hydrochloride
6 • (anti,anti)-2-(dimethylaminopyridin-3-ylmethyl)cyclohexylamine or its hydrochloride
7 • (anti,anti)-N-[2-(dimethylaminopyridin-3-ylmethyl)cyclohexyl]-2-fluorobenzamide or its hydrochloride
8 • (anti,anti)-2-chloro-N-[2-(dimethylaminopyridin-3-ylmethyl)cyclohexyl]benzamide or its hydrochloride
9 • (anti,anti)-N-[2-(dimethylaminopyridin-3-ylmethyl)cyclohexyl]-2-methylbenzamide or its hydrochloride
10 • (syn,syn)-N-[2-(dimethylaminopyridin-3-ylmethyl)cyclohexyl]-2-methylbenzamide or its hydrochloride
11 • (syn,syn)-N-[2-(dimethylaminopyridin-3-ylmethyl)cyclohexyl]acetamide or its hydrochloride
12 • (anti, anti) -N- [2- (dimethylaminopyridin-3-ylmethyl)cyclohexyl]acetamide or its hydrochloride
13 • (syn, syn) -N- [2-(dimethylaminophenylmethyl)cyclohexyl]-2-fluorobenzamide or its hydrochloride
14 • (syn, syn) -2-(dimethylaminophenylmethyl)cyclohexylamine or its hydrochloride
15 • (syn,syn)-N-[2-(dimethylamino-phenyl-methyl)-cyclohexyl]-acetamide or its hydrochloride
16 • (syn,syn)-N-[2-(dimethylamino-phenyl-methyl)-cyclohexyl]-benzamide or its hydrochloride
17 • (syn,syn)-2-chloro-N-[2-(dimethylamino-phenyl-methyl)-cyclohexyl]-benzamide or its hydrochloride
18 • (syn,syn)-N-[2-(dimethylamino-phenyl-methyl)-cyclohexyl]-2-methyl-benzamide or its hydrochloride
19 • (anti, anti) -N- [2- (dimethylamino-phenyl-methyl) - cyclohexyl]-acetamide or its hydrochloride
20 • (anti,anti)-2-(dimethylamino-phenyl-methyl)-cyclohexylamine or its hydrochloride
21 • (anti, anti) -N- [2- (dimethylamino-phenyl-methyl)-cyclohexyl]-benzamide or its hydrochloride
22 • (anti,anti)-N-[2-(dimethylamino-phenyl-methyl)-cyclohexyl]-2-methyl-benzamide or its hydrochloride
23 • (syn, syn)-2-chloro-N-{2-[(2-chloro-phenyl)-dimethylamino-methyl]-cyclohexyl}-benzamide or its hydrochloride
24 • (syn,syn)-2-[(2-chloro-phenyl)-dimethylaminomethyl]-cyclohexylamine or its hydrochloride
25 • (anti,anti)-2-chloro-N-{2-[(2-chloro-phenyl)-dimethylamino-methyl]-cyclohexyl}-benzamide or its hydrochloride
26 • (anti,anti)-2-[(2-chloro-phenyl)-dimethylaminomethyl]-cyclohexylamine or its hydrochloride
27 • (syn,syn)-N-{2-[(2-chloro-phenyl)-dimethylaminomethyl]-cyclohexyl}-2-fluoro-benzamide or its hydrochloride
28 • (anti,anti)-N-{2-[dimethylamino-(2-nitro-phenyl)-methyl]-cyclohexyl}-benzamide or its hydrochloride
29 • (anti,anti)-2-[dimethylamino-(2-nitro-phenyl)-methyl]-cyclohexylamine or its hydrochloride
30 • (anti,anti)-N-{2-[dimethylamino-(2-nitro-phenyl)-methyl]-cyclohexyl}-2-fluoro-benzamide or its hydrochloride
31 • (anti,anti)-2-chloro-N-{2-[dimethylamino-(2-nitro-phenyl)-methyl]-cyclohexyl}-benzamide or its hydrochloride
32 • (anti,anti)-N-{2-[dimethylamino-(2-nitro-phenyl)-methyl]-cyclohexyl}-2-methyl-benzamide or its hydrochloride

33 • (syn, syn) -N-{2- [dimethylamino- (2-nitro-phenyl) - methyl]-cyclohexyl}-acetamide or its hydrochloride
34 • (syn, syn) -N-2- [dimethylamino- (2-nitro-phenyl) - methyl]-cyclohexylamine or its hydrochloride
35 • (anti,anti)-N-{2-[(2-chloro-phenyl)-dimethylamino-methyl]-cyclohexyl}-acetamide or its hydrochloride
36 • (syn, anti)-2-(dimethylamino-phenyl-methyl)-cyclohexylamine
37 • (syn,anti)-N-[2-(dimethylamino-phenyl-methyl)-cyclohexyl]-benzamide
38 • (anti,anti)-N-{2-[dimethylamino-(2-methoxyphenyl)-methyl]-cyclohexyl}-benzamide
39 • (anti,anti)-N-{2-[dimethylamino-(2-nitro-phenyl)-methyl]-cyclohexyl}-benzamide
40 • (anti,anti)-N-{2-[(2-chloro-phenyl)-dimethylamino-methyl]-cyclohexyl}-benzamide
41 • (anti, anti)-N-{2-[dimethylamino-(2-methoxyphenyl)-methyl]-cyclohexyl}-acetamide
42 • (anti, anti)-2-[dimethylamino-(2-methoxy-phenyl)-methyl]-cyclohexylamine
43 • (anti,anti)-N-{2-[(2-chloro-phenyl)-dimethylamino-methyl]-cyclohexyl}-acetamide
44 • (anti,anti)-2-[(2-chloro-phenyl)-dimethylaminomethyl]-cyclohexylamine
45 • (anti,anti)-N-{2-[dimethylamino-(2-nitro-phenyl)-methyl]-cyclohexyl}-acetamide
46 • (anti,anti)-2-[dimethylamino-(2-nitro-phenyl)-methyl]-cyclohexylamine
47 • (syn,syn)-2-(dimethylamino-phenyl-methyl)-cyclohexylamine
48 • (syn,syn)-2-[(2-chloro-phenyl)-dimethylaminomethyl]-cyclohexylamine
49 • (anti,anti)-2-chloro-N-(3-dimethylamino-1-ethyl-2-methyl-3-phenyl-propyl)-benzamide
50 • (anti,anti)-3-dimethylamino-1-ethyl-2-methyl-3-phenyl-propylamine
51 • (syn,anti)-2-(dimethylamino-phenyl-methyl)-cyclohexyl-N-(n-propyl)-amine
52 • (syn,anti)-2-(morpholin-4-yl-phenyl-methyl)-cyclohexyl-N-(n-propyl)-amine
53 • (syn,anti)-2,N,N-trimethyl-1,3-diphenyl-N'-propyl-propane-1,3-diamine
54 • (syn, anti)-2-(dimethylamino-phenyl-methyl)-cyclohexyl-N-benzylamine
55 • (syn,anti)-2-(morpholin-4-yl-phenyl-methyl)-cyclohexyl-N-benzylamine
56 • (syn, anti)-2,N,N-trimethyl-1,3-diphenyl-N'-benzyl-propane-1,3-diamine
57 • (syn, anti)-2-(dimethylamino-phenyl-methyl)-cyclohexylamine
58 • (syn,anti)-2-(morpholin-4-yl-phenyl-methyl)-cyclohexylamine
59 • (syn,anti)-2,N,N-trimethyl-1,3-diphenyl-propane-1,3-diamine
60 • (syn,anti)-2-[(2-chlorophenyl)-dimethylaminomethyl]-cyclohexylamine
63 • (anti,anti)-2-[(2-chlorophenyl)-dimethylaminomethyl]-cyclohexylamine
62 • (syn,syn)-2-(dimethylamino-phenyl-methyl)-cyclohexylamine
63 • (anti,anti) -2- (dimethylamino-phenyl-methyl) - cyclohexylamine
64 • (syn,syn)-2-[(2-chlorophenyl)-dimethylaminomethyl]-cyclohexylamine
65 • (syn,syn)-2-(dimethylamino-pyridin-3-yl-methyl)-cyclohexylamine
66 • (anti,anti)-2-(dimethylamino-pyridin-3-yl-methyl)-cyclohexylamine
67 • (syn,syn)-2-(dimethylamino-(2-methoxyphenyl)-methyl)-cyclohexylamine
68 • (anti,anti)-2-(dimethylamino-(2-methoxyphenyl)-methyl)-cyclohexylamine
69 • (syn,syn)-2-(dimethylamino-(2-nitrophenyl)-methyl)-cyclohexylamine
70 • (anti,anti)-2-(dimethylamino-(2-nitrophenyl)-methyl)-cyclohexylamine.

**7.** Process for the preparation of a compound according to claim 1 of the general structure (I) or one of its pharmaceutically acceptable salts

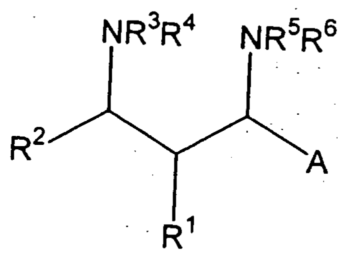

I ,

wherein

$R^1$ denotes methyl or ethyl,

$R^2$ denotes methyl, ethyl or phenyl, or
$R^1$ and $R^2$ together form - $(CH_2)_4$-;
$R^3$ denotes H, n-propyl or $-CH_2$-phenyl;
$R^4$ denotes H;
$R^5$ and $R^6$ each denote methyl or together form $-(CH_2)_2-O-(CH_2)_2-$; and
A denotes phenyl, 2-chlorophenyl, 2-methoxyphenyl, 2-nitrophenyl or pyridin-3-yl,

**characterized in that** an imine of the general structure (II)

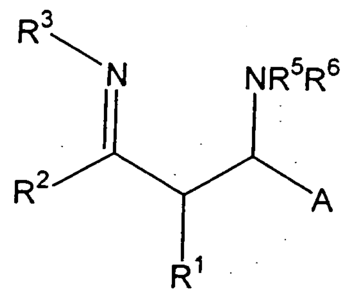

**II**

wherein $R^1$, $R^2$, $R^3$, $R^5$, $R^6$ and A are as defined above in this claim, is reacted with a suitable reducing agent.

8. Process according to claim 7, **characterized in that** the reducing agent is a complex hydride.

9. Process according to one of claims 7 or 8 for the diastereoselective preparation of a compound of the general structure (anti, anti-I) or one of its pharmaceutically acceptable salts

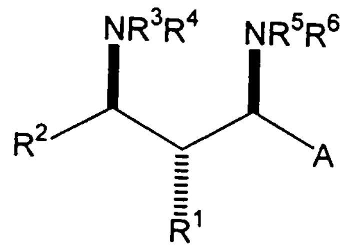

**anti,anti-I**

wherein $R^1$, $R^2$, $R^3$, $R^4$, $R^5$, $R^6$ and A are as defined in claim 7,
**characterized in that** an imine of the general structure (anti-II)

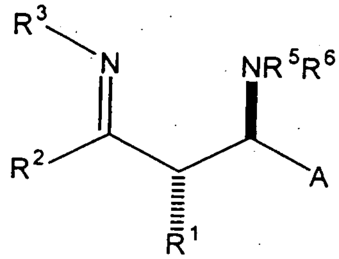

anti-II

wherein $R^1$, $R^2$, $R^3$, $R^5$, $R^6$ and A are as defined above in this claim,
is reacted with a suitable reducing agent, in particular zinc cyanoborohydride ($ZnCNBH_3$), $LiBH_4$, $NaBH_4$, $NaBH_3CN$ or $NaBH(OC(=O)CH_3)_3$, in an alcoholic solvent.

**10.** Process according to claim 9, **characterized in that** the reaction with zinc cyanoborohydride, $LiBH_4$, $NaBH_4$, $NaBH_3CN$ or $NaBH(OC(=O)CH_3)_3$ is carried out in methanol with warming from 0°C to room temperature over 8 to 24 h, preferably over 10 to 14 h.

**11.** Process according to one of claims 7 or 8 for the diastereoselective preparation of a compound of the general structure (syn,syn-I) or one of its pharmaceutically acceptable salts

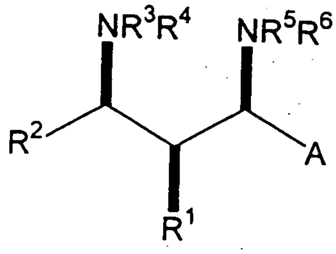

syn,syn-I ,

wherein $R^1$, $R^2$, $R^3$, $R^4$, $R^5$, $R^6$ and A are as defined in claim 7,
**characterized in that**
an imine of the general structure (anti-II)

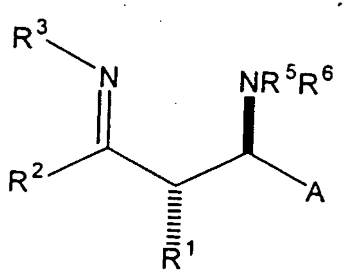

anti-II

wherein $R^1$, $R^2$, $R^3$, $R^5$, $R^6$ and A are as defined above in this claim,
is reacted with a suitable reducing agent, in particular L-Selectride or diisobutylaluminium hydride, in an ethereal

solvent.

**12.** Process according to claim 11, **characterized in that** the reaction is carried out with L-Selectride or diisobutylaluminium hydride in tetrahydrofuran with warming from 0°C to room temperature over 8 to 24 h, preferably over 10 to 14 h.

**13.** Process according to one of claims 7 to 12,
**characterized in that** the imine of the general structure (II) or (anti-II) is prepared by reaction of the Mannich base (III) or (anti-III)

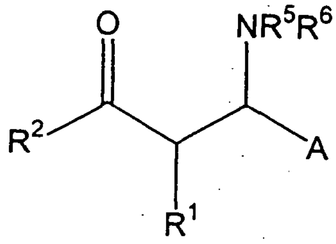

III

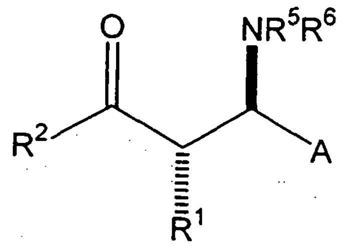

anti-III

wherein $R^1$, $R^2$, $R^5$, $R^6$ and A are as defined in claim 7, with an amine of the formula $R^3NH_2$, if $R^3$ in formula (II) or (anti-II) does not denote H, and with ammonium acetate, if $R^3$ in formula (II) or (anti-II) denotes H, in an ethereal or alcoholic solvent.

**14.** Process for the preparation of a compound of the general structure (I) or one of its pharmaceutically acceptable salts

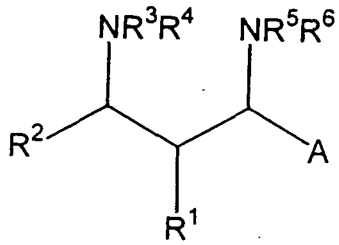

I

wherein

$R^1$ denotes methyl or ethyl,
$R^2$ denotes methyl, ethyl or phenyl, or
$R^1$ and $R^2$ together form - $(CH_2)_4$-;
$R^3$ and $R^4$ denote H;
$R^5$ and $R^6$ each denote methyl or together form -$(CH_2)_2$-O-$(CH_2)_2$-; and
A denotes phenyl, 2-chlorophenyl, 2-methoxyphenyl, 2-nitrophenyl or pyridin-3-yl,

**characterized by** the process steps:

(a) conversion of an amino-alcohol of the general structure (IV)

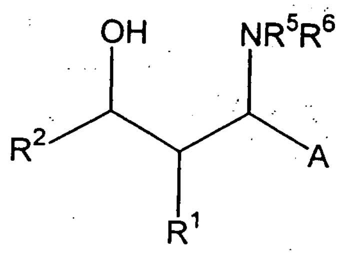

IV

,

wherein $R^1$, $R^2$, $R^5$, $R^6$ and A are as defined above in this claim, into the compound of the general structure (V)

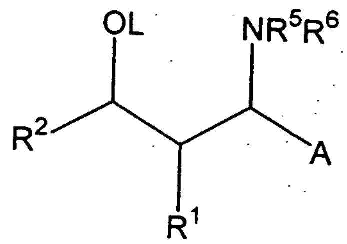

V

wherein L denotes mesyl or tosyl;
(b) conversion of the compound of the general structure (V) into an azide of the general structure (VI)

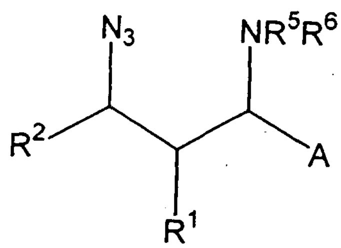

VI

and
(c) reduction of the azide of the general structure (VI) to the diamine of the general structure (I).

**15.** Process according to claim 14 for the diastereoselective preparation of a compound of the general structure (syn, anti-I) or (anti,anti-I) or one of its pharmaceutically acceptable salts

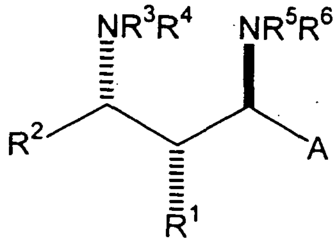
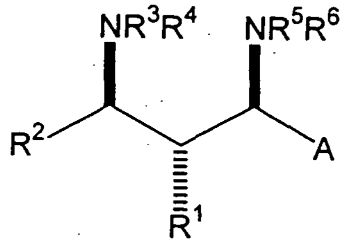

**syn,anti-I** **anti,anti-I** ,

wherein $R^1$, $R^2$, $R^3$, $R^4$, $R^5$, $R^6$ and A are as defined in claim 14, **characterized by** the process steps:

(a') conversion of an amino-alcohol of the general structure (anti,anti-IV) or (syn,anti-IV)

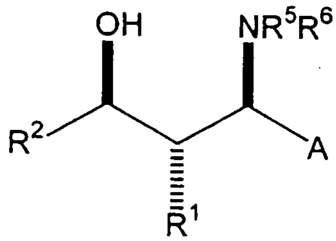
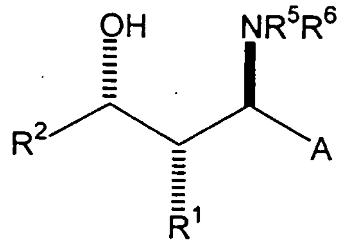

**anti,anti-IV** **syn,anti-IV** ,

wherein $R^1$, $R^2$, $R^5$, $R^6$ and A are as defined above in this claim, into the compound of the general structure (anti,anti-V) or (syn, anti-V)

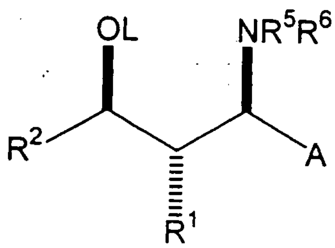
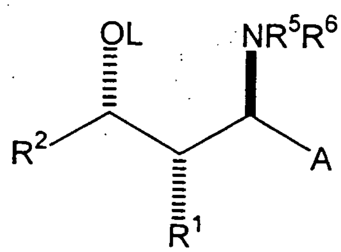

**anti,anti-V** **syn,anti-V**

wherein L denotes mesyl or tosyl;

(b') conversion of the compound of the general structure (anti,anti-V) or (syn,anti-V) into an azide of the general structure (syn,anti-VI) or (anti,anti-VI)

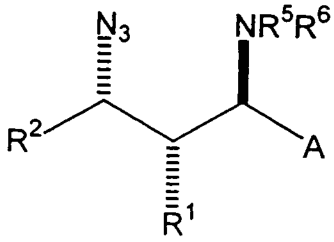

syn,anti-VI

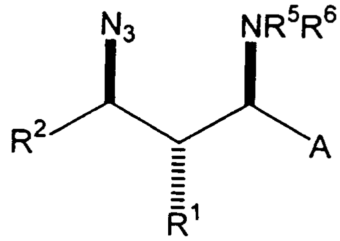

anti,anti-VI ;

and

(c') reduction of the azide of the general structure (syn,anti-VI) or (anti,anti-VI) to the diamine of the general structure (syn,anti-I) or (anti,anti-I).

**16.** Process for the preparation of a compound of the general structure (syn,anti-I) or one of its pharmaceutically acceptable salts

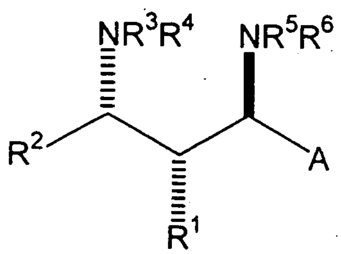

syn,anti-I ,

according to claim 2, wherein

$R^1$ denotes methyl or ethyl,
$R^2$ denotes methyl, ethyl or phenyl, or
$R^1$ and $R^2$ together form $-(CH_2)_4-$;
$R^3$ denotes H, n-propyl or $-CH_2$-phenyl;
$R^4$ denotes H;
$R^5$ and $R^6$ each denote methyl or together form $-(CH_2)_2-O-(CH_2)_2-$; and
A denotes phenyl, 2-chlorophenyl, 2-methoxyphenyl, 2-nitrophenyl or pyridin-3-yl,

**characterized by** the process steps

(aa) reaction of an imine of the general structure (VII)

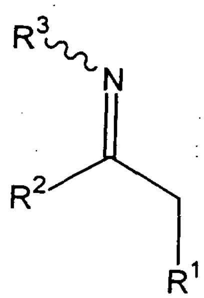

VII

wherein $R^1$, $R^2$ and $R^3$ are as defined above in this claim, with an iminium salt of the general structure (VIII)

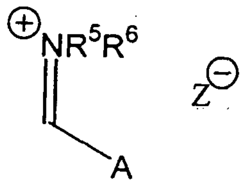

VIII

wherein $R^5$, $R^6$ and A are as defined above in this claim and $Z^\theta$ represents a suitable counter-ion; and
(bb) subsequent reduction.

**17.** Process according to claim 16 for the preparation of a compound of the general structure (syn,anti-I)

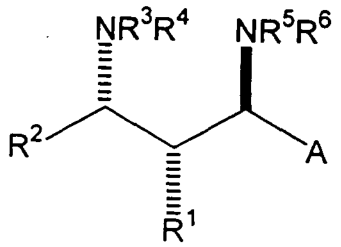

syn,anti-I

wherein $R^1$, $R^2$, $R^4$, $R^5$, $R^6$ and A are as defined in claim 16 and $R^3$ denotes H, **characterized in that** in a further process step (cc) a compound of the general structure (syn,anti-I), wherein $R^1$, $R^2$, $R^4$, $R^5$, $R^6$ and A are as defined in claim 16 and $R^3$ denotes -($CH_2$)-phenyl, wherein phenyl can be substituted by $C_{1-6}$-alkyl, is reacted with hydrogen ($H_2$) in the presence of a transition metal which is chosen from platinum, palladium and nickel.

**18.** Process for the preparation of a compound of the general structure (I) or one of its pharmaceutically acceptable salts

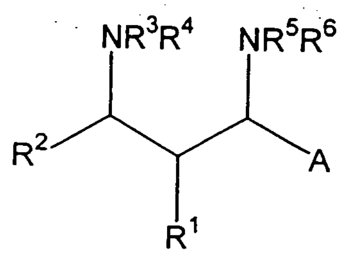

I

wherein

$R^1$ denotes methyl or ethyl,
$R^2$ denotes methyl, ethyl or phenyl, or
$R^1$ and $R^2$ together form $-(CH_2)_4-$;
$R^3$ denotes $C(=O)-R^7$;
$R^4$ denotes H;
$R^5$ and $R^6$ each denote methyl or together form - $(CH_2)_2-O-(CH_2)_2-$;
A denotes phenyl, 2-chlorophenyl, 2-methoxyphenyl, 2-nitrophenyl or pyridin-3-yl; and
$R^7$ denotes methyl, phenyl, 2-fluorophenyl, 2-chlorophenyl or 2-methylphenyl.

and which is present as a racemate or in the form of one or more diastereomers or one or more enantiomers, **characterized in that** a compound of the general structure (I) wherein $R^1$, $R^2$, $R^4$, $R^5$, $R^6$ and A are as defined above in this claim and $R^3$ denotes H and which is present as a racemate or in the form of one or more diastereomers or one or more enantiomers is reacted with an acylating reagent.

**19.** Process according to claim 18, **characterized in that** the acylating reagent is an acid chloride of the general formula $R^7-C(=O)-Cl$, wherein $R^7$ denotes methyl, phenyl, 2-fluorophenyl, 2-chlorophenyl or 2-methylphenyl.

**20.** Medicaments comprising a compound of the general structure (I) or one of its pharmaceutically acceptable salts, which are/is present as a racemate or in the form of one or more diastereomers or one or more enantiomers

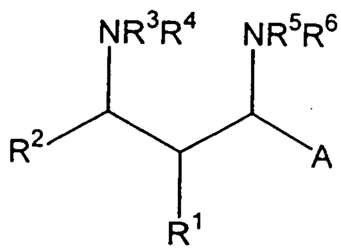

I

wherein

$R^1$ denotes methyl or ethyl,
$R^2$ denotes methyl, ethyl or phenyl, or
$R^1$ and $R^2$ together form $-(CH_2)_4-$;
$R^3$ denotes H, n-propyl , $-CH_2$-phenyl or $C(=O)-R^7$;
$R^4$ denotes H;
$R^5$ and $R^6$ each denote methyl or together form $-(CH_2)_2-O-(CH_2)_2-$;
A denotes phenyl, 2-chlorophenyl, 2-methoxyphenyl, 2-nitrophenyl or pyridin-3-yl; and
$R^7$ denotes methyl, phenyl, 2-fluorophenyl, 2-chlorophenyl or 2-methylphenyl.

**21.** Use of a compound of the general structure (I) or one of its pharmaceutically acceptable salts which are/is present as a racemate or in the form of one or more diastereomers or one or more enantiomers,

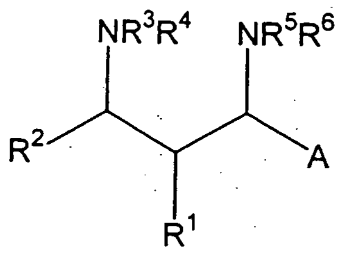

I

wherein

$R^1$ denotes methyl or ethyl,
$R^2$ denotes methyl, ethyl or phenyl, or
$R^1$ and $R^2$ together form $-(CH_2)_4-$;
$R^3$ denotes H, n-propyl, $-CH_2$-phenyl or $C(=O)-R^7$;
$R^4$ denotes H;
$R^5$ and $R^6$ each denote methyl or together form $-(CH_2)_2-O-(CH_2)_2-$;
A denotes phenyl, 2-chlorophenyl, 2-methoxyphenyl, 2-nitrophenyl or pyridin-3-yl; and
$R^7$ denotes methyl, phenyl, 2-fluorophenyl, 2-chlorophenyl or 2-methylphenyl,

for the preparation of a medicament for treatment and/or prophylaxis of pain.

**22.** Use of a compound of the general structure (I) or one of its pharmaceutically acceptable salts which are/is present as a racemate in the form of one or more diastereomers or one or more enantiomers

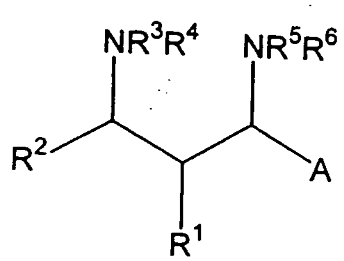

I ,

wherein

$R^1$ denotes methyl or ethyl,
$R^2$ denotes methyl, ethyl or phenyl, or
$R^1$ and $R^2$ together form $-(CH_2)_4-$;
$R^3$ denotes H, n-propyl, $-CH_2$-phenyl or $C(=O)-R^7$;
$R^4$ denotes H;
$R^5$ and $R^6$ each denote methyl or together form $-(CH_2)_2-O-(CH_2)_2-$;
A denotes phenyl, 2-chlorophenyl, 2-methoxyphenyl, 2-nitrophenyl or pyridin-3-yl ; and
$R^7$ denotes methyl, phenyl, 2-fluorophenyl, 2-chlorophenyl or 2-methylphenyl,

for the preparation of a medicament for treatment and/or prophylaxis of urinary incontinence, itching, tinnitus aurium

and/or diarrhoea.

## Revendications

**1.** Composé de formule générale (I) ou l'un de ses sels pharmaceutiquement acceptables

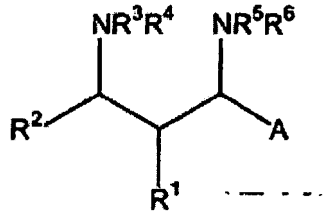

I ,

formule dans laquelle

$R^1$ désigne un reste méthyle ou éthyle,
$R^2$ désigne un reste méthyle, éthyle ou phényle,

ou bien

$R^1$ et $R^2$ forment ensemble un groupe $-(CH_2)_4-$ ;
$R^3$ représente H, un reste n-propyle, $-CH_2$-phényle ou C (=O) $-R^7$ ;
$R^4$ représente H ;
$R^5$ et $R^6$ représentent chacun un reste méthyle ou forment ensemble un groupe $-(CH_2)_2-O-(CH_2)_2-$ ;
A désigne un reste phényle, 2-chlorophényle, 2-méthoxyphényle, 2-nitrophényle ou pyridine-3-yle ; et
$R^7$ désigne un reste méthyle, phényle, 2-fluorophényle, 2-chlorophényle ou 2-méthylphényle,

excepté

- la benzyl-[2-diméthylamino-phényl-méthyl)-cyclohexyl]-amine
- la (2-méthyl-1,3-diphényl-3-pipéridine-1-yl-propyl)-propyl-amine.

**2.** Composé suivant la revendication 1 ou l'un de ses sels pharmaceutiquement acceptables, le composé de formule générale (I) étant présent sous forme du diastéréoisomère de formule (syn,anti-I)

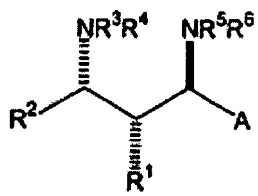

syn,anti-I

et étant le cas échéant énantiomériquement pur.

**3.** Composé suivant la revendication 1 ou l'un de ses sels pharmaceutiquement acceptables, le composé de formule générale (I) étant présent sous forme de diastéréoisomères de formule (anti,anti-I)

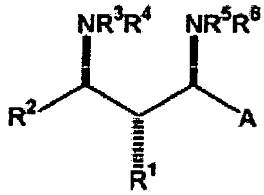

anti,anti-I

et étant le cas échéant énantiomériquement pur.

4. Composé suivant la revendication 1 ou l'un de ses sels pharmaceutiquement acceptables, le composé de formule générale (I) étant présent sous forme de diastéréoisomères de formule (anti,syn-I)

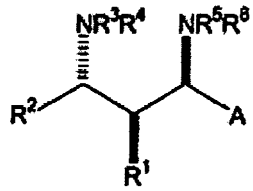

anti,syn-I

et étant le cas échéant énantiomériquement pur.

5. Composé suivant la revendication 1 ou l'un de ses sels pharmaceutiquement acceptables, le composé de formule générale (I) étant présent sous forme de diastéréoisomères de formule (syn,syn-I)

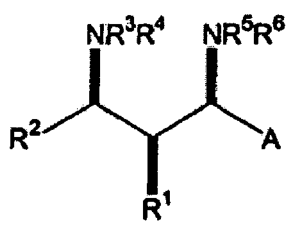

syn,syn-I

et étant le cas échéant énantiomériquement pur.

6. Composé suivant la revendication 1 ou l'un de ses sels pharmaceutiquement acceptables, choisi entre les composés suivants :

- (syn,syn)-N-[2-(diméthylaminopyridine-3-ylméthyl)-cyclohexyl]benzamide ou son chlorhydrate
- (syn,syn)-2-(diméthylaminopyridine-3-ylméthyl)-cyclohexylamine ou son chlorhydrate
- (syn,syn)-N-[2-(diméthylaminopyridine-3-ylméthyl)-cyclohexyl]-2-fluorobenzamide ou son chlorhydrate
- (syn,syn)-2-chloro-N-[2-(diméthylaminopyridine-3-ylméthyl)cyclohexyl]-benzamide ou son chlorhydrate
- (anti,anti)-N-[2-(diméthylaminopyridine-3-yl-méthyl)cyclohexyl]benzamide ou son chlorhydrate
- (anti,anti)-2-(diméthylaminopyridine-3-ylméthyl)-cyclohexylamine ou son chlorhydrate
- (anti,anti)-N-[2-(diméthylaminopyridine-3-yl-méthyl)cyclohexyl]-2-fluorobenzamide ou son chlorhydrate
- (anti,anti)-2-chloro-N-[2-(diméthylaminopyridine-3-ylméthyl)cyclohexyl]benzamide ou son chlorhydrate
- (anti,anti)-N-[2-(diméthylaminopyridine-3-yl-méthyl)cyclohexyl]-2-méthylbenzamide ou son chlorhydrate

- (syn,syn)-N-[2-(diméthylaminopyridine-3-ylméthyl)-cyclohexyl]-2-méthylbenzamide ou son chlorhydrate
- (syn,syn)-N-[2-(diméthylaminopyridine-3-ylméthyl)-cyclohexyl]acétamide ou son chlorhydrate
- (anti,anti)-N-[2-(diméthylaminopyridine-3-yl-méthyl)cyclohexyl]acétamide ou son chlorhydrate
- (syn,syn)-N-[2-diméthylaminophénylméthyl)-cyclohexyl]-2-fluorobenzamide ou son chlorhydrate
- (syn,syn)-2-(diméthylaminophénylméthyl)-cyclohexylamine ou son chlorhydrate
- (syn,syn)-N-[2-(diméthylamino-phényl-méthyl)-cyclohexyl]acétamide ou son chlorhydrate
- (syn,syn)-N-[2-(diméthylamino-phényl-méthyl)-cyclohexyl]benzamide ou son chlorhydrate
- (syn,syn)-2-chloro-N-[2-(diméthylamino-phénylméthyl)cyclohexyl]-benzamide ou son chlorhydrate
- (syn,syn)-N-[2-(diméthylamino-phényl-méthyl)-cyclohexyl]-2-méthyl-benzamide ou son chlorhydrate
- (anti,anti)-N-[2-(diméthylamino-phényl-méthyl)-cyclohexyl]acétamide ou son chlorhydrate
- (anti,anti)-2-(diméthylamino-phényl-méthyl)-cyclohexylamine ou son chlorhydrate
- (anti,anti)-N-[2-(diméthylamino-phényl-méthyl)-cyclohexyl]benzamide ou son chlorhydrate
- (anti,anti)-N-[2-(diméthylamino-phényl-méthyl)-cyclohexyl]-2-méthyl-benzamide ou son chlorhydrate
- (syn,syn)-2-chloro-N-{2-[(2-chloro-phényl)-diméthylamino-méthyl]cyclohexyl}-benzamide ou son chlorhydrate
- (syn,syn)-2-[(2-chloro-phényl)-diméthylaminométhyl]cyclohexylamine ou son chlorhydrate
- (anti,anti)-2-chloro-N-{2-[(2-chloro-phényl)-diméthylamino-méthyl]-cyclohexyl}-benzamide ou son chlorhydrate
- (anti,anti)-2-[(2-chloro-phényl)-diméthylaminométhyl]cyclohexylamine ou son chlorhydrate
- (syn,syn)-N-{2-[(2-chloro-phényl)-diméthylaminométhyl]-cyclohexyl}-2-fluoro-benzamide ou son chlorhydrate
- (anti,anti)-N-{2-[diméthylamino-(2-nitro-phényl)-méthyl]cyclohexyl}-benzamide ou son chlorhydrate
- (anti,anti)-2-[diméthylamino-(2-nitro-phényl)-méthyl]cyclohexylamine ou son chlorhydrate
- (anti,anti)-N-{2-[diméthylamino-(2-nitro-phényl)-méthyl]-cyclohexyl}-2-fluoro-benzamide ou son chlorhydrate
- (anti,anti)-2-chloro-N-{2-[diméthylamino-(2-nitro-phényl)-méthyl]cyclohexyl}-benzamide ou son chlorhydrate
- (anti,anti)-N-{2-[diméthylamino-(2-nitro-phényl)-méthyl]-cyclohexyl}-2-méthyl-benzamide ou son chlorhydrate
- (syn,syn)-N-{2-[diméthylamino-(2-nitro-phényl)-méthyl]cyclohexyl}-acétamide ou son chlorhydrate
- (syn,syn)-N-2-[diméthylamino-(2-nitro-phényl)-méthyl]cyclohexylamine ou son chlorhydrate
- (anti,anti)-N-{2-[(2-chloro-phényl)-diméthylamino-méthyl]cyclohexyl}-acétamide ou son chlorhydrate
- (syn,anti)-2-(diméthylamino-phényl-méthyl)-cyclohexylamine
- (syn,anti)-N-[2-(diméthylamino-phényl-méthyl)-cyclohexyl]benzamide
- (anti,anti)-N-{2-[diméthylamino-(2-méthoxyphényl)-méthyl]cyclohexyl}-benzamide
- (anti,anti)-N-{2-[diméthylamino-(2-nitro-phényl)-méthyl]cyclohexyl}-benzamide
- (anti,anti)-N-{2-[(2-chloro-phényl)-diméthylamino-méthyl]cyclohexyl}-benzamide
- (anti,anti)-N-{2-[diméthylamino-(2-méthoxyphényl)-méthyl]cyclohexyl}-acétamide
- (anti,anti)-2-[diméthylamino-(2-méthoxy-phényl)-méthyl]cyclohexylamine
- (anti,anti)-N-{2-[(2-chloro-phényl)-diméthylamino-méthyl]cyclohexyl}-acétamide
- (anti,anti)-2-[(2-chloro-phényl)-diméthylaminométhyl]cyclohexylamine
- (anti,anti)-N-2-[diméthylamino-(2-nitro-phényl)-méthyl]cyclohexyl}-acétamide
- (anti,anti)-2-[diméthylamino-(2-nitro-phényl)-méthyl]cyclohexyl}-acétamide
- (syn,syn)-2-(diméthylamino-phényl-méthyl)-cyclohexylamine
- (syn,syn)-2-[(2-chloro-phényl)-diméthylaminométhyl)cyclohexylamine
- (anti,anti)-2-chloro-N-(3-diméthylamino-1-éthyl-2-méthyl-3-phénylpropyl)-benzamide
- (anti,anti)-3-diméthylamino-1-éthyl-2-méthyl-3-phényl-propylamine
- (syn,anti)-2-(diméthylamino-phényl-méthyl)-cyclohexyl-N-(n-propyl)-amine
- (syn,anti)-2-(morpholine-4-yl-phényl-méthyl)-cyclohexyl-N-(n-propyl)-amine
- (syn,anti)-2-N,N-triméthyl-1,3-diphényl-N'-propyl-propane-1,3-diamine
- (syn,anti)-2-(diméthylamino-phényl-méthyl)-cyclohexyl-N-benzylamine
- (syn,anti)-2-(morpholine-4-yl-phényl-méthyl)-cyclohexyl-N-benzylamine
- (syn,anti)-2,N,N-triméthyl-1,3-diphényl-N'-benzyl-propane-1,3-diamine
- (syn,anti)-2-(diméthylamino-phényl-méthyl)-cyclohexylamine
- (syn,anti)-2-(morpholine-4-yl-phényl-méthyl)-cyclohexylamine
- (syn,anti)-2,N,N-triméthyl-1,3-diphényl-propane-1,3-diamine
- (syn,anti)-2-[(2-chlorophényl)-diméthylaminométhyl)cyclohexylamine
- (anti,anti)-2-[(2-chlorophényl)-diméthylaminométhyl]cyclohexylamine
- (syn,syn)-2-(diméthylamino-phényl-méthyl)-cyclohexylamine
- (anti,anti)-2-(diméthylamino-phényl-méthyl)-cyclohexylamine
- (syn,syn)-2-[(2-chlorophényl)-diméthylaminométhyl]cyclohexylamine
- (syn,syn)-2-(diméthylamino-pyridine-3-yl-méthyl)-cyclohexylamine
- (anti,anti)-2-(diméthylamino-pyridine-3-yl-méthyl)-cyclohexylamine

- (syn,syn)-2-(diméthylamino-(2-méthoxyphényl)-méthyl)cyclohexylamine
- (anti,anti)-2-(diméthylamino-(2-méthoxyphényl)-méthyl)cyclohexylamine
- (syn,syn)-2-(diméthylamino-(2-nitrophényl)-méthyl)cyclohexylamine
- (anti,anti)-2-(diméthylamino-(2-nitrophényl)-méthyl)cyclohexylamine

**7.** Procédé de production d'un composé suivant la revendication 1, de formule générale (I) ou de l'un de ses sels pharmaceutiquement acceptables

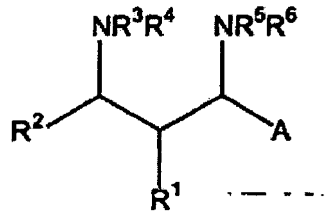

I ,

formule dans laquelle

$R^1$ désigne un reste méthyle ou éthyle,
$R^2$ désigne un reste méthyle, éthyle ou phényle,

ou bien

$R^1$ et $R^2$ forment ensemble un groupe $-(CH_2)-_4$ ;
$R^3$ représente H, un reste n-propyle ou $-CH_2$-phényle ;
$R^4$ représente H ;
$R^5$ et $R^6$ représentent chacun un reste méthyle ou forment ensemble un groupe $-(CH_2)_2-O-(CH_2)_2-$ ; et
A désigne un reste phényle, 2-chlorophényle, 2-méthoxyphényle, 2-nitrophényle ou pyridine-3-yle,

**caractérisé en ce qu'**on fait réagir une imine de formule générale (II)

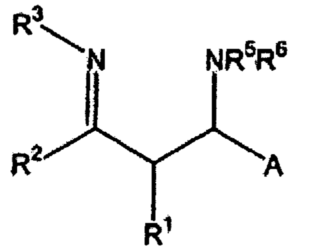

II ,

dans laquelle $R^1$, $R^2$, $R^3$, $R^5$, $R^6$ et A sont tels que définis ci-dessus dans cette revendication, avec un agent réducteur convenable.

**8.** Procédé suivant la revendication 7, **caractérisé en ce que** l'agent réducteur est un hydrure complexe.

**9.** Procédé suivant l'une des revendications 7 et 8 pour la production diastéréosélective d'un composé de formule générale (anti,anti-I) ou de l'un de ses sels pharmaceutiquement acceptables,

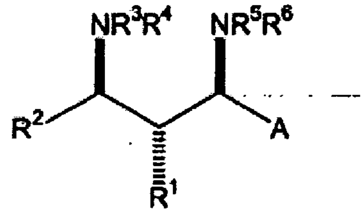

**anti,anti-I** ,

formule dans laquelle $R^1$, $R^2$, $R^3$, $R^4$, $R^5$, $R^6$ et A sont tels que définis dans la revendication 7, **caractérisé en ce qu'**on fait réagir l'imine de formule générale (anti-II)

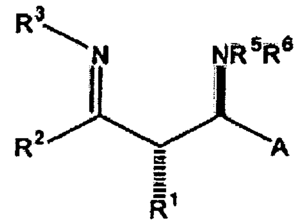

**anti-II** ,

dans laquelle $R^1$, $R^2$, $R^3$, $R^5$, $R^6$ et A sont tels que définis ci-dessus dans cette revendication, avec un agent réducteur convenable, en particulier le cyanoborohydrure de zinc ($ZnCNBH_3$), $LiBH_4$, $NaBH_4$, $NaBH_3CN$ ou $NaBH(OC(=O)CH_3)_3$ dans un solvant alcoolique .

**10.** Procédé suivant la revendication 9, **caractérisé en ce que** la réaction avec le cyanoborohydrure de zinc, $LiBH_4$, $NaBH_4$, $NaBH_3CN$ ou $NaBH(OC(=O)CH_3)_3$ est conduite dans du méthanol avec réchauffement à une température allant de 0°C à la température ambiante en une période de 8 à 24 heures, avantageusement en une période de 10 à 14 heures.

**11.** Procédé suivant l'une des revendications 7 et 8 pour la production diastéréosélective d'un composé de formule générale (syn,syn-I) ou de l'un de ses sels pharmaceutiquement acceptables

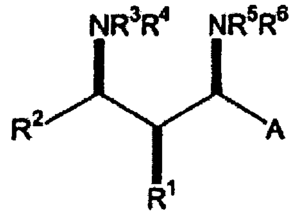

**syn,syn-I** ,

formule dans laquelle
$R^1$, $R^2$, $R^3$, $R^4$, $R^5$, $R^6$ et A sont définis comme dans la revendication 7,
**caractérisé en ce qu'**on fait réagir une imine de formule générale (anti-II)

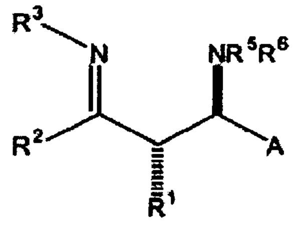

**anti-II** .

dans laquelle $R^1$, $R^2$, $R^3$, $R^5$, $R^6$ et A sont tels que définis ci-dessus dans cette revendication, avec un agent réducteur convenable, en particulier le L-Selectride ou l'hydrure de diisobutylaluminium, dans un solvant du type d'un éther.

**12.** Procédé suivant la revendication 11, **caractérisé en ce que** la réaction avec le L-Selectride ou avec l'hydrure de diisobutylaluminium est conduite dans du tétrahydrofuranne avec réchauffement de 0°C à la température ambiante en une période de 8 à 24 heures, avantageusement de 10 à 14 heures.

**13.** Procédé suivant l'une des revendications 7 à 12, **caractérisé en ce que** l'imine de formule générale (I) ou (anti-II) est préparée par réaction de la base de Mannich (III) ou respectivement (anti-III)

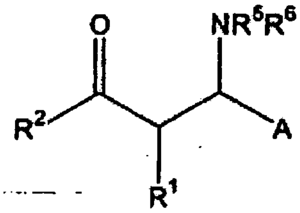

**III**

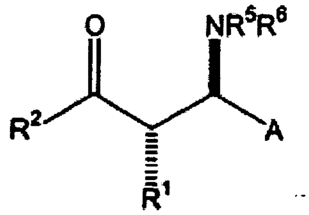

**anti-III**

où $R^1$, $R^2$, $R^5$, $R^6$ et A sont tels que définis dans la revendication 7, avec une amine de formule $R^3NH_2$, lorsque $R^3$ dans la formule (II) ou (anti-II) ne représente pas H, et avec l'acétate d'ammonium lorsque $R^3$ dans la formule (II) ou (anti-II) représente H, dans un solvant du type d'un éther ou d'un alcool.

**14.** Procédé de production d'un composé de formule générale (I) ou de l'un de ses sels pharmaceutiquement acceptables

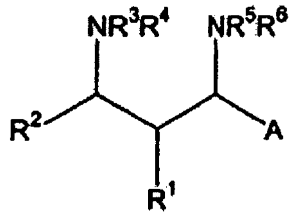

**I** ,

dans laquelle

$R^1$ est un reste méthyle ou éthyle,
$R^2$ est un reste méthyle, éthyle ou phényle,

ou bien

$R^1$ et $R^2$ forment ensemble un groupe $-(CH_2)_4-$ ;
$R^3$ et $R^9$ représentent H ;
$R^5$ et $R^6$ représentent chacun un reste méthyle ou forment ensemble un reste $-(CH_2)_2-O(CH_2)_2-$ ; et
A est un reste phényle, 2-chlorophényle, 2-méthoxyphényle, 2-nitrophényle ou pyridine-3-yle,

**caractérisé par** les étapes suivantes :

(a) transformation d'un aminoalcool de formule générale (IV)

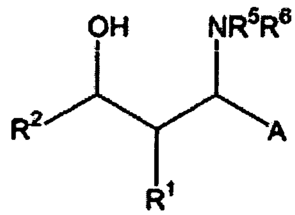

IV ,

dans laquelle $R^1$, $R^2$, $R^5$, $R^6$ et A sont tels que définis ci-dessus pour cette revendication,
en un composé de formule générale (V)

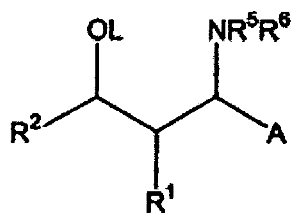

V ,

dans laquelle L est un reste mésyle ou tosyle ;
(b) transformation du composé de formule générale (V) en un azide de formule générale (VI)

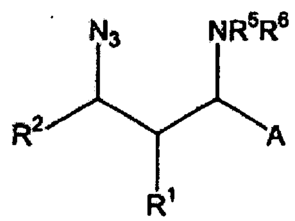

VI ;

et
(c) réduction de l'azide de formule générale (VI) en la diamine de formule générale (I).

**15.** Procédé suivant la revendication 14 pour la production diastéréosélective d'un composé de formule générale (syn, anti-I) ou (anti,anti-I) ou de l'un de ses sels pharmaceutiquement acceptables,

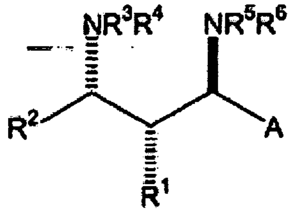 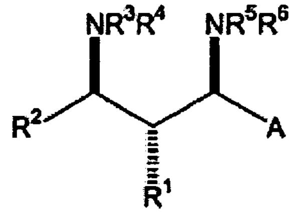

**syn,anti-I** **anti,anti-I** ,

formules dans lesquelles $R^1$, $R^2$, $R^3$, $R^4$, $R^5$, $R^6$ et A sont tels que définis dans la revendication 14, **caractérisé par** les étapes suivantes :

(a') transformation d'un aminoalcool de formule générale (anti,anti-IV) ou (syn,anti-IV)

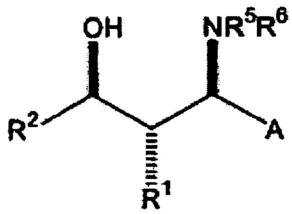 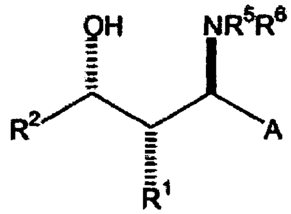

**anti,anti-IV** **syn,anti-IV** ,

formules dans lesquelles $R^1$, $R^2$, $R^5$, $R^6$ et A sont tels que définis ci-dessus dans cette revendication, en composé de formule générale (anti,anti-V) ou respectivement (syn,anti-V)

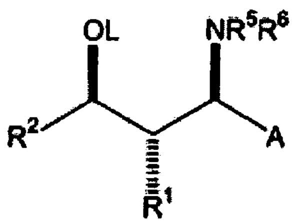 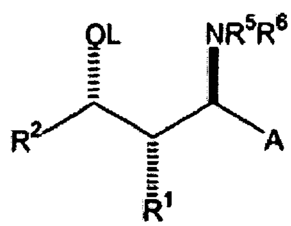

**anti,anti-V** **syn,anti-V**

où L désigne un reste mésyle ou tosyle ;

(b') transformation du composé de formule générale (anti,anti-V) ou (syn,anti-V) en un azide de formule générale (syn,anti-VI) ou respectivement (anti,anti-VI)

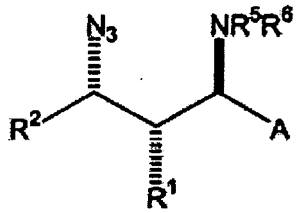

syn,anti-VI

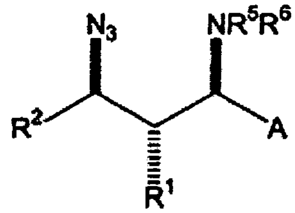

anti,anti-VI ;

et

(c') réduction de l'azide de formule générale (syn,anti-VI) ou (anti,anti-VI) en la diamine de formule générale (syn,anti-I) ou respectivement (anti,anti-I).

**16.** Procédé de production d'un composé de formule générale (syn,anti-I) ou de l'un de ses sels pharmaceutiquement acceptables,

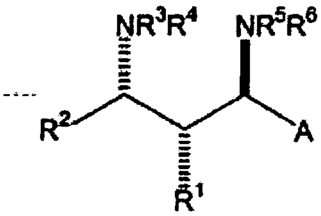

syn,anti-I ,

selon la revendication 2,
formule dans laquelle

$R^1$ est un reste méthyle ou éthyle,
$R^2$ est un reste méthyle, éthyle, ou phényle,

ou bien

$R^1$ et $R^2$ forment ensemble un groupe $-(CH_2)_4-$ ;
$R^3$ représente H, un reste n-propyle ou $-CH_2$-phényle ;
$R^4$ représente H ;
$R^5$ et $R^6$ désignent chacun un reste méthyle ou forment ensemble un groupe$-(CH_2)_2-O(CH_2)_2-$ , et
A est un reste phényle, 2-chlorophényle, 2-méthoxyphényle, 2-nitrophényle ou pyridine-3-yle,

**caractérisé par** les étapes suivantes

(aa) réaction d'une imine de formule générale (VII)

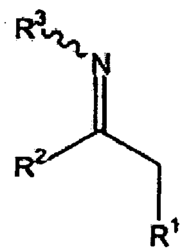

VII ,

dans laquelle $R^1$, $R^2$ et $R^3$ sont tels que définis ci-dessus dans cette revendication, avec un sel d'iminium de formule générale (VIII)

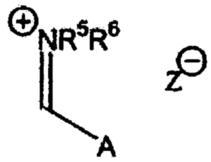

VIII ;

dans laquelle $R^5$, $R^6$ et A sont tels que définis ci-dessus dans cette revendication et $Z^\ominus$ désigne un ion complémentaire convenable ; et
(bb) réduction subséquente.

**17.** Procédé suivant la revendication 16 pour la production d'un composé de formule générale (syn,anti-I)

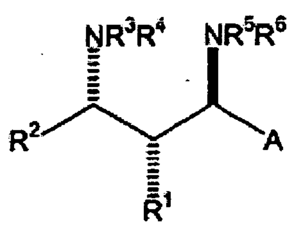

syn,anti-I ,

dans laquelle $R^1$, $R^2$, $R^4$, $R^5$, $R^6$ et A sont tels que définis dans la revendication 16 et $R^3$ représente H, **caractérisé en ce que** dans une autre étape (cc), on fait réagir un composé de formule générale (syn,anti-I) dans laquelle $R^1$, $R^2$, $R^4$, $R^5$, $R^6$ et A sont tels que définis dans la revendication 16 et $R^3$ est un reste $-(CH_2)$-phényle, dont la portion phényle peut être substituée par un radical alkyle en $C_1$ à $C_6$, en présence d'un métal de transition qui est choisi entre le platine, le palladium et le nickel, avec de l'hydrogène ($H_2$).

**18.** Procédé de production d'un composé de formule générale (I) ou de l'un de ses sels pharmaceutiquement acceptables,

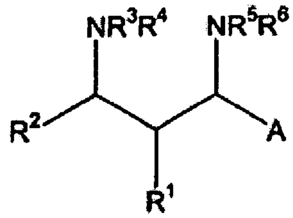

I ,

dans laquelle

$R^1$ est un reste méthyle ou éthyle,
$R^2$ est un reste méthyle, éthyle ou phényle,

ou bien

$R^1$ et $R^2$ forment ensemble un groupe $-(CH_2)_4-$;
$R^3$ est un groupe C(=O)-$R^7$;
$R^4$ représente H ;
$R^5$ et $R^6$ désignent chacun un reste méthyle ou forment ensemble un groupe $-(CH_2)_2-O(CH_2)_2-$;
A est un reste phényle, 2-chlorophényle, 2-méthoxyphényle, 2-nitrophényle ou pyridine-3-yle ; et
$R^7$ désigne un reste méthyle, phényle, 2-fluorophényle, 2-chlorophényle ou 2-méthylphényle,

et qui se présente sous forme de racémate, sous forme d'un
ou plusieurs diastéréoisomères ou sous forme d'un ou plusieurs énantiomères,
**caractérisé en qu'**on fait réagir un composé de formule générale (I), dans laquelle $R^1$, $R^2$, $R^4$, $R^5$, $R^6$ et A sont tels que définis ci-dessus dans cette revendication et $R^3$ représente H, et qui se présente sous forme de racémate, sous forme d'un ou plusieurs diastéréoisomères ou sous forme d'un ou plusieurs énantiomères, avec un réactif d'acylation.

**19.** Procédé suivant la revendication 18, **caractérisé en ce que** le réactif d'acylation est un chlorure d'acide de formule générale $R^7$-C(=O)-Cl dans laquelle $R^7$ est un reste méthyle, phényle, 2-fluorophényle, 2-chlorophényle ou 2-méthylphényle.

**20.** Médicament contenant un composé de formule générale (I) ou l'un de ses sels pharmaceutiquement acceptables, qui se présente sous forme de racémate, sous forme d'un ou plusieurs diastéréoisomères ou d'un ou plusieurs énantiomères,

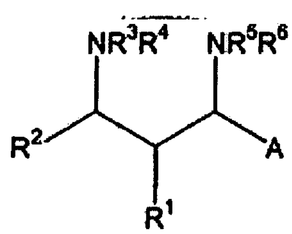

I ,

formule dans laquelle

$R^1$ est un reste méthyle ou éthyle,
$R^2$ est un reste méthyle, éthyle ou phényle,

ou bien

$R^1$ et $R^2$ forment ensemble un groupe - $(CH_2)_4$- ;
$R^3$ représente H, un reste n-propyle, -$CH_2$-phényle ou C(=O)-$R^7$;
$R^4$ représente H ;
$R^5$ et $R^6$ représentent chacun un reste méthyle ou forment ensemble un groupe -$(CH_2)_2$-O$(CH_2)_2$- ;
A est un reste phényle, 2-chlorophényle, 2-méthoxyphényle, 2-nitrophényle ou pyridine-3-yle ; et
$R^7$ est un reste méthyle, phényle, 2-fluorophényle, 2-chlorophényle ou 2-méthylphényle,

**21.** Utilisation d'un composé de formule générale (I) ou de l'un de ses sels pharmaceutiquement acceptables, qui se présente sous forme de racémate, sous forme d'un ou plusieurs diastéréoisomères ou sous forme d'un ou plusieurs énantiomères,

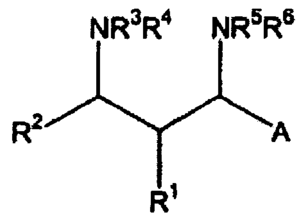

I ,

formule dans laquelle

$R^1$ est un reste méthyle ou éthyle,
$R^2$ est un reste méthyle, éthyle ou phényle,

ou bien

$R^1$ et $R^2$ forment ensemble un groupe -$(CH_2)_4$- ;
$R^3$ représente H, un reste n-propyle, -$CH_2$-phényle ou C(=O)-$R^7$ ;
$R^4$ représente H ;
$R^5$ et $R^6$ représentent chacun un reste méthyle ou forment ensemble un groupe -$(CH_2)_2$-O$(CH_2)_2$- ;
A est un reste phényle, 2-chlorophényle, 2-méthoxyphényle, 2-nitrophényle ou pyridine-3-yle ; et
$R^7$ est un reste méthyle, phényle, 2-fluorophényle, 2-chlorophényle ou 2-méthylphényle,

pour la préparation d'un médicament destiné à la thérapie et/ou à la prophylaxie de la douleur.

**22.** Utilisation d'un composé de formule générale (I) ou de l'un de ses sels pharmaceutiquement acceptables, qui se présente sous forme de racémate, sous forme d'un ou plusieurs diastéréoisomères ou d'un ou plusieurs énantiomères,

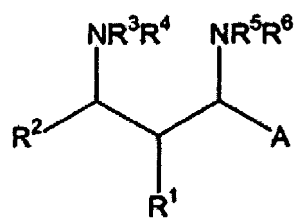

I ,

formule dans laquelle

$R^1$ est un reste méthyle ou éthyle,
$R^2$ est un reste méthyle, éthyle ou phényle,

ou bien

$R^1$ et $R^2$ forment ensemble un groupe $-(CH_2)_4-$;
$R^3$ représente H, un reste n-propyle, $-CH_2$-phényle ou C(=O)-$R^7$;
$R^4$ représente H ;
$R^5$ et $R^6$ représentent chacun un reste méthyle ou forment ensemble un groupe $-(CH_2)_2-O(CH_2)_2-$ ;
A est un reste phényle, 2-chlorophényle, 2-méthoxyphényle, 2-nitrophényle ou pyridine-3-yle ; et
$R^7$ est un reste méthyle, phényle, 2-fluorophényle, 2-chlorophényle ou 2-méthylphényle,

pour la préparation d'un médicament destiné à la thérapie et/ou à la prophylaxie de l'incontinence d'urine, du prurit, des acouphènes et/ou de la diarrhée.